# EUROPEAN PATENT SPECIFICATION

(11) **EP 3 721 867 B1**
(45) Date of publication and mention of the grant of the patent: **05.01.2022**
(21) Application number: 20170365.9
(22) Date of filing: 17.01.2017
(51) Int. Cl.: A61K 8/898, A61Q 5/12, A61Q 19/00, C08G 77/26

(54) **SILYLATED AMINO ACID/SILANE COPOLYMER AS EMULSIFIER OR POWDER DISPERSANT**
SILYLIERTES AMINOSÄURE/SILAN-COPOLYMER ALS EMULGATOR ODER PULVERDISPERGIERMITTEL
COPOLYMÈRE D'ACIDE AMINÉ SILYLÉ/SILANE COMME ÉMULSIFIANT OU DISPERSANT DE POUDRE

(30) Priority: 27.01.2016 JP 2016013507
(43) Date of publication of application: 14.10.2020
(62) Divisional of application: 17744022.9
(73) Proprietor: Seiwa Kasei Company, Limited, Higashiosaka-shi, Osaka 579-8004 (JP)
(72) Inventor: YOSHIOKA, Masato, Osaka 579-8004 (JP); HOMMA, Yuta, Osaka 579-8004 (JP); TOMIHISA, Shota, Osaka 579-8004 (JP)
(74) Representative: Vossius & Partner Patentanwälte Rechtsanwälte mbB

(56) References cited:
- EP-A2- 0 934 966
- EP-A2- 1 477 160
- WO-A1-03/078503

## Description

### TECHNICAL FIELD

The disclosure provided herein relates to a cosmetic substrate composed of a silylated amino acid/silane compound copolymer obtained by polycondensing at least one silylated amine acid having two or more hydroxyl groups bonding directly to a silicon atom and at least one silane compound generating two or more hydroxyl groups bonding directly to a silicon atom by hydrolysis and to a cosmetic containing the cosmetic substrate, being capable to imparting lustrous gloss, good sliding effect and non-sticky moisturizing feeling to hair, being excellent in a split hair preventing effect, being capable of imparting smoothness and non-sticky moist feeling to skin, the colpolymers having a function as an emulsifier and a powder dispersant. The invention is defined by the uses illustrated in the appended claims.

### BACKGROUND ART

It has conventionally been tried to blend a silicone oil in cosmetics for hair and skin, to impart gloss and luster and to impart water repellency owing to the silicone oil. However, a silicone oil is intrinsically a hydrophobic substance and thus has a problem that when being applied to hair, the silicone oil hardly adheres to hairs damaged and having hydrophilized surface and the effect thereof is not exerted. Further, since a silicone oil is not easily blended in an aqueous cosmetic, it is necessary to concurrently use a surfactant (emulsifier) in the aqueous cosmetic for keeping stability of the product.

An amino-modified silicone obtained by introducing an amino functional group into a silicone for enhancing adsorptive powder thereof to hair and skin (Patent Document 1, etc.) and a polyether-modified silicone endowed with hydrophilicity by introducing a polyoxyalkylene group into a silicone for providing easy blending into a hydrophilic cosmetic and for imparting a moisturizing property (Patent Document 2) have been used. However, the moisturizing property imparting effect is not obtained only by addition of an amino group, and even if the polyether-modified silicone is used, the aspect of adsorbability to hair and skin is not satisfactory though the degree of stability in an aqueous cosmetic increases. See also EP 0 934 966 A2.

Further, there is also an attempt of introducing an amino acid into an amino-modified silicone to impart a moisturizing property of the amino acid. However, for introducing an amino acid into a silicone chain, the reaction needs to be conducted in an organic solvent at high temperature (Patent Document 3) or needs to use α-amino acid-N-carboxylic acid anhydride (Patent Document 4). Thus, the production is not easy.

The present inventors have developed a silylated peptide made by introducing a silane compound having two or more hydroxyl groups bonding directly to a silicon atom into a peptide obtained by hydrolyzing a protein, and developed a silylated peptide/silane compound copolymer composition obtained by polycondensing the silylated peptide with a silane compound generating two or more hydroxyl groups bonding directly to a silicon atom by hydrolysis (Patent Document 5). The present inventors have attempted to blend this in a cosmetic to impart a moisturizing property owing to the peptide and luster-gloss and sliding effect owing to the silicone to hair and skin. This silylated peptide/silane compound copolymer composition has a nature as an emulsifier, and particularly, has been used as an emulsifier for producing an O/W type emulsion(Patent Document 6).

The protein hydrolysate used for obtaining the silylated peptide, however, has little adsorption site to weakly acidic hair and skin because of small content of basic amino acids in the protein, and is not sufficiently satisfactory regarding imparting luster and gloss, excluding extremely special protein sources. Depending on the protein hydrolysate to be used, the moisturizing effect is too high to impart sticky feeling to hair and skin in some cases. Further, depending on the protein source for the peptide and the extent of degradation thereon, the hydrolysate contained in a cosmetic is associated, to generate insoluble matters. That is, a problem of stability of the cosmetic exists. For this reason, the extent of degradation of a protein and the reaction ratio thereof in copolymerizing with a silane compound should be investigated sufficiently, for producing a more stable product of a silylated peptide/silane compound copolymer composition.

### PRIOR ART REFERENCES

### PATENT DOCUMENTS

(Patent Document 1) JP H06-080536A
(Patent Document 2) JP H08-188519A
(Patent Document 3) JP2009-540040A
(Patent Document 4) JP2002-145724A
(Patent Document 5) JP2000-007795A
(Patent Document 6) JP2001-048732A

### SUMMARY OF THE INVENTION

### PROBLEMS TO BE SOLVED BY THE INVENTION

The present disclosure has an object of providing a cosmetic substrate composed of a silicone-based polymer compound, being excellent in adsorbability to hair and skin, being capable of imparting lustrous gloss, good sliding effect and non-sticky moisturizing feeling to hair, being excellent in a split hair preventing effect, being capable of imparting smoothness and non-sticky moist feeling to skin, and having also a function as an emulsifier.

### MEANS FOR SOLVING THE PROBLEMS

The present inventors have intensively studied to solve the above-described problem and resultantly found that a silylated amino acid/silane compound copolymer obtained by using a silylated amino acid instead of a silylated peptide can be produced easily in an aqueous solvent, and further, when blended in a cosmetic, the copolymer can impart lustrous gloss, good sliding effect, non-sticky moisturizing feeling and a split hair preventing effect to hair and can impart smoothness and non-sticky moist feeling to skin, and further, has also a function as an emulsifier and a powder dispersant, leading to completion of the present invention.

A first aspect of the disclosure is a cosmetic substrate composed of a silylated amino acid/silane compound copolymer having a structural unit U represented by the following general formula (Ia), (Ib) or (Ic): 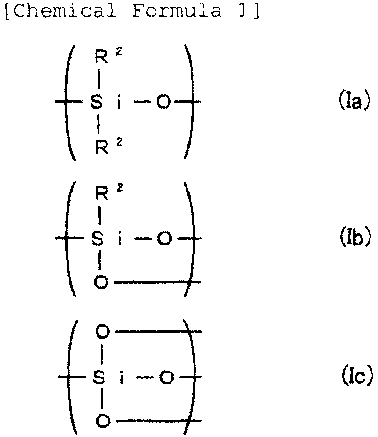 [wherein, R² represents a hydroxyl group, a phenyl group, or an alkyl group having 1 to 20 carbon atoms optionally containing nitrogen, sulfur, a halogen or a phenyl group in the group, and the groups R² may be the same or different.] and a structural unit W represented by the following general formula (Id) or (Ie): 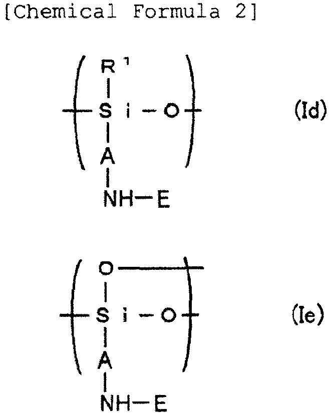 [wherein, R¹ represents a hydroxyl group or an alkyl group having 1 to 3 carbon atoms, the groups R¹ may be the same or different, A is a divalent group bonding Si and N, and represents at least one group selected from the group consisting of R^{B}, *R^{B}OCH₂CH (OH) CH₂, *R^{B}S, *R^{B}NH and *R^{B}OCOCH₂CH₂ (R^{B} represents an alkyl group having 1 to 5 carbon atoms, and * represents a side bonding to Si), E represents a residue obtained by removing one primary amino group from an α amino acid, and when E has an amino group other than the α amino group, N in the above-described other amino group may be bended to A of the other structural unit W], wherein the molar ratio of structural unit W:structural unit U is in the range of 1:5 to 1:150 (Claim 1). This cosmetic substrate can impart lustrous gloss, good sliding effect, combability, non-sticky moisturizing feeling and a split hair preventing effect to hair, and can impart smoothness and non-sticky moist feeling to skin.

The residue represented by E includes any of residues obtained by removing an α amino group from an α amino acid and residues obtained by removing one amino group other than an α amino group from an α amino acid. The α amino group denotes an amino group bonding directly to a carbon bonding directly to a carboxyl group of an α amino acid. The case having an amino group other than an α amino group is a case in which the α amino acid is a basic amino acid, and the other amino group is an amino group bonded to the side chain of the α amino acid. "When E has an amino group other than an α amino group, N of the above-described other amino group is bonded to A of the other structural unit W" means that a structure represented by the following formula (If) is formed, or the like.

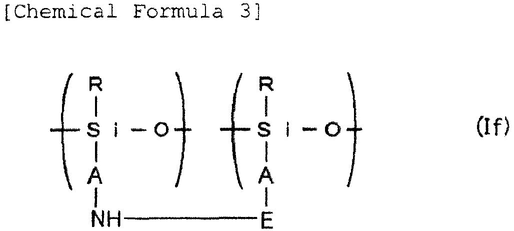

E in the formula (If) represents an α amino group-removed residue obtained by removing an α amino group from an α amino acid, and N of an amino group of the side chain contained in the E is bonded to A. R represents R¹ or 0-.

As a more preferable embodiment of the above-described first aspect, the disclosure also provides a cosmetic substrate composed of a silylated amino acid/silane compound copolymer in which the structural unit U is represented by (Ia) or (Ib) and the structural unit W is represented by (Id) (Claim 2). According to this aspect the silylated amino acid/silane compound copolymer does not get too high viscosity, and can be easily used as a cosmetic substrate.

The effect capable of imparting gloss, good sliding effect, moisturizing feeling, a split hair preventing effect and the like to hair and imparting smoothness and moist feeling to skin of the above-described cosmetic substrate improves more when basic amino acids are contained in the above-described α amino acid (an α amino acid used as a raw material for producing the above-described silylated amino acid/silane compound copolymer, and represented by the formula NH₂-E) and its proportion is higher. By including of basic amino acids in the above-described α amino acid, the silylated amino acid/silane compound copolymer scarcely causes phenomena such as agglomeration and precipitation even in an aqueous cosmetic, and stability in the cosmetic increases. As a preferable aspect, a cosmetic substrate characterized in that the above-described α amino acid contains basic amino acids (Claim 3) is provided.

The above-described effect by inclusion of basic amino acids in the above-described α amino acid becomes clear from around where the proportion of basic amino acids exceeds 35 mol%, and becomes remarkable when the proportion is 40 mol% or more. Thus, as a more preferable embodiment of the above-described first embodiment, a cosmetic substrate characterized in that 40 mol% or more of the above-described α amino acid are basic amino acids (Claim 4) is provided.

Of basic amino acids, arginine is best in the action of imparting moisturizing feeling and moist feeling to hair and skin. Thus, as a further preferable embodiment of the above-described first embodiment, a cosmetic substrate in which 40 mol% of more of the above-described α amino acid are composed of arginine (Claim 5) is provided.

As a further aspect the disclosure provides a cosmetic substrate characterized in that a group represented by the following general formula (II) is bonded to the end of the above-described silylated amino acid/silane compound copolymer (Claim 6). 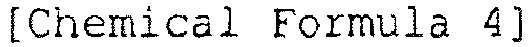 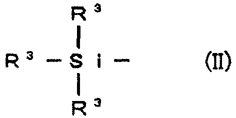 [wherein, R³ represents an alkyl group having 1 to 4 carbon atoms or a phenyl group, and the groups R³ may be the same or different.]

According to this aspect, a hydroxyl group remaining at the end of the silylated amino acid/silane compound copolymer is bonded to a group represented by the general formula (II). As a result, long-term storage stability of the above-described copolymer in a cosmetic increases, and a cosmetic showing little change during long-term storage can be obtained.

A further aspect is a cosmetic substrate composed of a silylated amino acid/silane compound copolymer obtained by poly-condensing at least one silylated amino acid in which a silyl group represented by the following general formula (III): 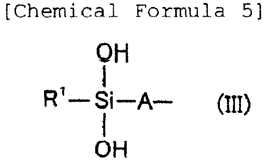 [wherein, R¹ represents a hydroxyl group or an alkyl group having 1 to 3 carbon atoms, A is a divalent group bonding Si and N, and represents a group selected from the groups consisting of R^{B}, *R^{B}OCH₂CH (OH) CH₂, *R^{B}S, *R^{B}NH and *R^{B}OCOCH₂CH₂ (R^{B} represents an alkyl group having 1 to 5 carbon atoms, and * represents a side bonded to Si)] is bonded to an α amino group of an α amino acid, and at least one silane compound represented by the following general formula (IV):

R²ₙSi(OH)ₚY₍₄₋ₚ₋ₙ₎ (IV)

[wherein, R² represents a hydroxyl group, a phenyl group, or an alkyl group having 1 to 20 carbon atoms optionally containing nitrogen, sulfur, a halogen or a phenyl group in the group, n is an integer of 0 to 2, and n R²s may be the same or different. p is an integer of 2 to 4, n+p ≤ 4, and (4-p-n) Ys represent an alkoxy group having 1 to 6 carbon atoms or a hydrogen atom],
wherein the reaction molar ratio of silylated amino acid:silane compound is in the range of 1:5 to 1:150 (Claim 7) .

This cosmetic substrate is obtained by defining the above-described cosmetic substrate of the first aspect by way of the production method of the copolymer. Therefore, this cosmetic substrate can impart lustrous gloss, good sliding effect, combability, non-sticky moisturizing feeling and a split hair preventing effect to hair and can impart smoothness and non-sticky moist feeling to skin, likewise. When an α amino acid has an amino group other than an α amino group (that is, when an amino acid as the raw material contains basic amino acids), a silyl group represented by the above-described general formula (III) may be bonded to all or a part of the above-described other amino acids (amino acids in the side chain) in addition to an α amino group.

As further aspect the disclosure provides a cosmetic substrate composed of a silylated amino acid/silane compound copolymer obtained by poly-condensing the silylated amino acid and the silane compound, then, further reacting a silane compound represented by the following general formula (VIII):

R³₃Si-OH (VIII)

[wherein, three R³s represent an alkyl group having 1 to 4 carbon atoms or a phenyl group, and the three R³s may be the same or different] (Claim 8). This cosmetic substrate is obtained by defining the above-described cosmetic substrate as a preferable embodiment of the first embodiment by way of the production method of the copolymer. According to this embodiment, a hydroxyl group remaining at the end of the silylated amino acid/silane compound copolymer of the second embodiment reacts with a silane compound represented by the general formula (VIII). As a result, long-term storage stability of the above-described copolymer in a cosmetic increases, and a cosmetic showing little change during long-term storage can be obtained.

Further aspect is a cosmetic containing the above-described cosmetic substrate according to the first or second aspect. The content of the above-described silylated amino acid/silane compound copolymer in the cosmetic is suitably about 0.01% by mass to 20% by mass in the cosmetic for manifesting lustrous gloss, good sliding effect, non-sticky moisturizing feeling and a split hair preventing effect on hair and manifesting smoothness and non-sticky moist feeling on skin, though there is a slight difference depending on the kind and the form of a cosmetic. Then, a cosmetic containing 0.01% by mass or more and 20% by mass or less of the above-described cosmetic substrate according to the first embodiment or the second embodiment (Claim 9) is provided.

### EFFECT OF THE INVENTION

The cosmetic substrate of the present disclosure composed of a silylated amino acid/silane compound copolymer can be produced more easily as compared with a silylated peptide/silane compound copolymer composition of a conventional product, and furthermore, the silylated amino acid/silane compound copolymer produced can impart lustrous gloss, good sliding effect, non-sticky moisturizing feeling and a split hair preventing effect to hair and can impart smoothness and non-sticky moist feeling to skin. Claimed is according to the invention the use of these compositions as emulsifier and a powder dispersant. Further, the cosmetic blended with the above-described cosmetic substrate imparts the effects as described above to hair and skin, and additionally, storage stability of the cosmetic substrate is good even in an aqueous cosmetic.

### BRIEF DESCRIPTION OF THE DRAWINGS

[FIG. 1] Fig. 1 shows an infrared absorption spectra of the silylated amino acid/silane compound copolymer produced in Example 1.
[FIG. 2] Fig. 2 shows an infrared absorption spectra of the silylated amino acid/silane compound copolymer produced in Example 5.
[FIG. 3] Fig. 3 shows an infrared absorption spectra of the silylated amino acid/silane compound copolymer produced in Example 6.

### MODES FOR CARRYING OUT THE INVENTION

The silylated amino acid as the raw material of the cosmetic substrate of the present invention, polycondensation of a silylated amino acid and a silane compound as a step of producing the cosmetic substrate of the present invention, the reaction of the polycondensate and a silane compound having three alkyl groups bonded to a silicon atom, and the cosmetic containing the cosmetic substrate of the present invention will be illustrated below in this order.

### [Silylated amino acid]

The silylated amino acid is obtained by reacting a silane coupling agent generating two or more hydroxyl groups bonding directly to a silicon atom with an α amino group of an α amino acid. As the α amino acid used in production of the silylated amino acid, those used for cosmetics can be used and are not particularly restricted. Any of acidic amino acids such as aspartic acid, glutamic acid and the like, neutral amino acids such as glycine, alanine, serine, threonine, methionine, cysteine, valine, leucine, isoleucine, phenylalanine, tyrosine, proline, tryptophan, asparagine, glutamine and the like, basic amino acids such as arginine, lysine, histidine, ornithine and the like can be used.

The silane coupling agent generating two or more hydroxyl groups bonding directly to a silicon atom includes, for example, 3-glycidoxypropylmethyldimethoxysilane, 3-glycidoxypropylmethyldiethoxysilane, 3-methacryloxypropylmethyldimethoxysilane, 3-methacryloxypropylmethyldiethoxysilane, N-(2'-aminoethyl)-3-aminopropylmethyldiethoxysilane, 3-glycidoxypropyltrimethoxysilane, 3-glycidoxypropyltriethoxysilane and the like, and commercially available products of any compounds can be used. For example, KBM-402, KBE-402, KBM-502, KBE-503, KBM-403 and KBE-403 (all are trade names) manufactured by Shin-Etsu Chemical Co., Ltd., SH6040, SZ6023 and SZ6030 (all are trade names) manufactured by Dow Corning Toray Co., Ltd., and the like correspond to them.

The silylated amino acid can be produced by methods described in JP-A H8-59424 and JP-A H8-67608. For example, a silane coupling agent having two or more alkoxy groups bonding directly to a silicon atom is dropped into an amino acid aqueous solution while stirring with heat under a basic condition and both the compounds are allowed to contact, as a result, the silane coupling agent is bonded to an amino group of the amino acid, to obtain a silylated amino acid having two or more hydroxyl groups generated on a silicon atom as represented by the following general formula (VI). 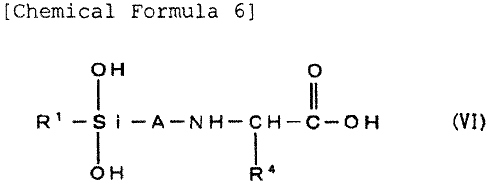 [wherein, R¹ and A are the same as in the general formula (I), and R⁴ represents the side chain of an amino acid.]

When the α amino acid is a basic amino acid and a silane compound generating two or more hydroxyl groups bonding directly to a silicon atom is bonded also to an amino group of the side chain, the copolymer can be represented by the following general formula (VII): 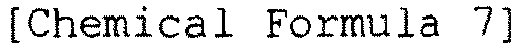 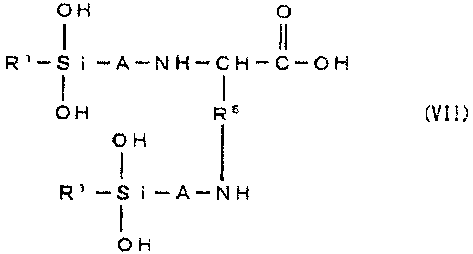 [wherein, R¹ and A are the same as in the general formula (I), and R⁵ represents a side chain excluding an amino group in the side chain of a basic amino acid.].

In the reaction of introducing a silyl group into an amino group in producing a silylated amino acid, a silane coupling agent and an α amino acid are reacted under basic condition of the solution having pH of 9 to 11, and if a silane coupling agent is dropped into the solution having pH in this range, the alkoxy group bonding directly to a silicon atom is hydrolyzed and converted into a hydroxyl group. That is, in the reaction of an α amino acid and a silane coupling agent, it is not necessary to previously hydrolyze a silane coupling agent to cause conversion into a hydroxyl group, and the reaction can be performed by adding a silane coupling agent directly to an amino acid aqueous solution having pH controlled to 9 to 11.

As the α amino acid used in the reaction, a single amino acid may be used or an amino acid mixture may be used. When an amino acid mixture is used, the present amount of each amino acid in the copolymer is unclear in producing the silylated amino acid/silane compound copolymer, since reactivity to a silane coupling agent varies slightly depending on the kind of the amino acid. For this reason, it is desirable that silylation is conducted using a single amino acid, in producing a silylated amino acid.

Progress of the silylation reaction can be confirmed by measuring the amino nitrogen amount in the reaction liquid by a Van Slyke method. The reaction product is, after adjusting the concentration, subjected to the subsequent polycondensation reaction with a silane compound. The reaction product is usually a mixture of a silylated amino acid and an unreacted α amino acid, and a mixture having a content of a silylated amino acid of 40 mol% or more is preferable as one which is subjected to a polycondensation reaction with a silane compound. Therefore, if the mixture itself after completion of the silylation reaction is used in a polycondensation reaction, it is preferable that the reaction rate of the silylation reaction is 40% or more. Further, the reaction liquid may be neutralized, and concentrated when appropriate, and purified with an ion exchange resin, a dialysis membrane, electrodialysis, ultrafiltration and the like, before being used as the raw material in polycondensation with a silane compound.

### [Polycondensation of silylated amino acid and silane compound]

Next, the silylated amino acid obtained as described above and a silane compound generating two or more hydroxyl groups bonding directly to a silicon atom represented by the general formula (IV):

R²ₙSi(OH)ₚY₍₄₋ₚ₋ₙ₎ (IV)

[wherein, R² represents a hydroxyl group, a phenyl group, or an alkyl group having 1 to 20 carbon atoms optionally containing nitrogen, sulfur, a halogen or a phenyl group in the group, n is an integer of 0 to 2, and n R²-s may be the same or different. p is an integer of 2 to 4, n+p ≤ 4, and (4-p-n) Y represent an alkoxy group having 1 to 6 carbon atoms or a hydrogen atom.] are poly-condensed, and the silane compound having two or more hydroxyl groups bonding directly to a silicon atom is obtained by hydrolyzing a silane compound represented by the following general formula (V):

R²ₙSiX₍₄₋ₙ₎ (V)

[wherein, R² and n are the same as in the above-described general formula (IV), and n R²s may be the same or different. (4-n) Xs represent at least one group selected from the group consisting of a hydroxyl group, an alkoxy group and a halogen group.]. In the hydrolysis, an alkoxy group or a halogen group of the silane compound can be hydrolyzed to be converted into a hydroxyl group, by dropping into an aqueous solution having pH adjusted to an acidic side of 2 to 3 with hydrochloric acid, sulfuric acid and the like or into an aqueous solution having pH adjusted to 10 to 11 with a sodium hydroxide aqueous solution, a potassium hydroxide aqueous solution and the like. R² in the general formula (Ia), (Ib), (IV) or (V) is preferably a hydroxyl group, a phenyl group or an alkyl group having 4 to 14 carbon atoms.

Examples of a silane compound represented by the general formula (V) generating two or more hydroxyl groups bonding directly to a silicon atom by hydrolysis include tetramethoxysilane, methyltrimethoxysilane, methyldimethoxysilane, dimethyldimethoxysilane, phenyltrimethoxysilane, diphenyldimethoxysilane, hexyltrimethoxysilane, decyltrimethoxysilane, vinyltrimethoxysilane, 3-methacryloxypropyltrimethoxysilane, 3-methacryloxypropylmethyldimethoxysilane, N-(2-aminoethyl)-3-aminopropyltrimethoxysilane, N-(2-aminoethyl)-3-aminopropylmethyldimethoxysilane, 3-aminopropyltrimethoxysilane, N-phenyl-3-aminopropyltrimethoxysilane, 3-chrolopropyltrimethoxysilane, 3-chroloopropylmethyldimethoxysilane, 3-mercaptopropyltrimethoxysilane, tetraethoxysilane, methyltriethoxysilane, methyldiethoxysilane, dimethyldiethoxysilane, phenyltriethoxysilane, diphenyldiethoxysilane, hexyltriethoxysilane, octyltriethoxysilane, viyltriethoxysilane, 3-methacryloxypropyltriethoxysilane, 3-methacryloxypropylmethyldiethoxysilane, N-(2-aminoethyl)-3-aminopropylmethyldiethoxysilane, 3-aminopropyltriethoxysilane, 3-chrolopropyltriethoxysilane, 3-chrolopropylmethyldiethoxysilane, 3-glycidoxypropylmethyldiethoxysilane, 3-isocyanatepropyltriethoxysilane, methyldichrolosilane, methyltrichrolosilane, dimethyldichrolosilane, phenyltrichrolosilane, diphenyldichrolosilane, vinyltrichrolosilane, and 3-chrolopropylmetyldichrolosilane. These silane compounds generate two or more hydroxyl groups bonding directly to a silicon atom by hydrolysis to yield a silane compound represented by the general formula (IV).

Commercially available products of the silane compound represented by the general formula (V) can be used. For example, KBM-13, KBM-22, KBM-103, KBM-3063, KBM-3033, KBM-1003, KBM-502, KBM-603, KBM-602, KBM-903, KBM-573, KBM-803, KBM-403, KBE-13, KBE-22, KBE-103, KBE-1003, KBE-402, KBE-502, KBE-503, KBE-1003, KBE-603, KBE-602, KBE-3033, KBE-3083, KBE-903 (all are trade names) manufactured by Shin-Etsu Chemical Co., Ltd., Z-6366, Z-6329, Z-6013, Z-6383, Z-6321, Z-6265, Z-6275, Z-6403, Z-6583, Z-6586, Z-6187, Z-6341, Z-6210, Z-6124, ACS-8 (all are trade names) manufactured by Dow Corning Toray Co., Ltd., and the like can be mentioned.

Polycondensation of a silylated amino acid with a silane compound represented by the general formula (IV) can be carried out by reference to a production method of a silylated peptide/silane compound copolymer composition described in JP-H11-286550A or Patent Document 6. Specifically, a silylated amino acid aqueous solution is adjusted to an acidic pH of 2 to 3 with hydrochloric acid, sulfuric acid and the like or adjusted to a basic pH of 10 to 11 with a sodium hydroxide aqueous solution, a potassium hydroxide aqueous solution and the like, and a silane compound represented by the general formula (V) is dropped therein, by this, an alkoxy group, a halogen atom and the like of the silane compound are hydrolyzed and converted into a hydroxyl group, to give a form represented by the general formula (IV). Then, a hydroxyl group of the silylated amino acid and a hydroxyl group of the silane compound itself are poly-condensed and polymerized.

The reaction molar ratio of the silylated amino acid to the silane compound represented by the general formula (V), that is, the silylated amino acid:silane compound is in the range of 1:5 to 1:150. The silylated amino acid:silane compound is preferably 1:20 to 1:150, more preferably 1:20 to 1:100. When reacted in the range of 1:5 to 1:150, the amino acid amount in the silylated amino acid/silane compound copolymer is approximately 1% by mass to 20% by mass, though it varies slightly since there is a difference in the molecular weight depending on the functional group of the silane compound used in the reaction. When the amino acid presence amount in the silylated amino acid/silane compound copolymer is less than 1% by mass, the nature of the silicone appears strongly, oily feeling due to a silicone chain increases, and adhesion to hydrophilic hairs becomes difficult. As a result, there is a possibility that lustrous gloss, good sliding effect, non-sticky moisturizing feeling and a split hair preventing effect cannot be imparted sufficiently to hair and smoothness and non-sticky moist feeling cannot be imparted to skin. When the amino acid presence amount in the silylated amino acid/silane compound copolymer exceeds 20% by mass, there is a possibility of generating sticky feeling when applied to hair and skin.

If the viscosity of the generated silylated amino acid/silane compound copolymer is extremely high, blending into a cosmetic becomes difficult and handling thereof becomes worse. Thus, a silylated amino acid/silane compound copolymer, of which aqueous solution having the solid content of 75% shows a viscosity in the range of 500 to 20,000 mPa·s at 20°C, is preferable. For producing a silylated amino acid/silane compound copolymer having the viscosity within this range, it is necessary that the reaction molar ratio of the silylated amino acid to the silane compound, that is, the silylated amino acid:silane compound is in the range of 1:5 to 1:150.

The silylated amino acid used for producing the silylated amino acid/silane compound copolymer may be a single silylated amino acid or a mixture obtained by mixing several silylated amino acids. It is preferable that the silylated amino acid contains a basic amino acid from the standpoint of the action of imparting moisturizing feeling and moist feeling to hair and skin when blended into a cosmetic. Particularly, when the amount of all amino acids is 100 mol%, it is preferable that the basic amino acid content is 40 mol% or more. Of silylated basic amino acids, silylated arginine manifests the effect of imparting moisturizing feeling and moist feeling more successfully and is preferably used.

The temperature of the reaction of a silylated amino acid and a silane compound is preferably 30°C to 60°C since when it is too low, the reaction is not carried out easily, while when too high, an alkoxy group and a halogen group of a silane compound represented by the general formula (V) are hydrolyzed rapidly. The reaction time varies depending on the reaction amount and the reaction temperature, and it is preferable that the above-described silane compound represented by the general formula (V) is dropped over a period of 30 minutes to 2 hours, and thereafter, stirring of the reaction liquid is continued for 1 to 24 hours. After completion of the reaction, an alkali aqueous solution such as a dilute sodium hydroxide solution and the like is added in the case of acidic side of the solution and an acid aqueous solution such as dilute hydrochloric acid, dilute sulfuric acid and the like is added in the case of basic side, and the mixture is stirred to neutralize the solution. Since an increase in the molecular weight progresses by neutralization, stirring is continues for about 1 to 24 hours to complete the reaction after neutralization.

### [Reaction with silane compound having three alkyl groups bonding to silicon atom]

The silylated amino acid/silane compound copolymer obtained as described above can be used as it is as a cosmetic substrate. However, since a hydroxyl group remains at the end silyl group of the copolymer, there is a possibility that the silylated amino acid/silane compound copolymers agglomerate mutually to increase the molecular weight. For this reason, it is preferable for the silylated amino acid/silane compound copolymer obtained above that a silane compound represented by the following general formula (VIII):

R³₃Si-OH (VIII)

[wherein, three R³s represent an alkyl group having 1 to 4 carbon atoms or a phenyl group, and the three R³s may be the same or different] is further added to block a hydroxyl group on the silylated amino acid/silane compound copolymer.

The above-described silane compound having one hydroxyl group represented by the general formula (VIII) is obtained, for example, by hydrolyzing a silane compound represented by the following general formula (IX):

R³₃Si-R⁴ (IX)

[wherein, R³ is the same as described above, and R⁴ represents an alkoxy group having 1 to 6 carbon atoms or a halogen atom.].

The silane compound represented by the general formula (IX) generating one hydroxyl group bonding directly to a silicon atom by hydrolysis includes, for example, dimethylvinylchlorosilane, n-butyldimethylchlorosilane, tert-butyldimethylchlorosilane, tert-butyldiphenylchlorosilane, octadecyldimethylchlorosilane, methyldiphenylchlorosilane, tri-n-butylchlorosilane, triethylchlorosilane, trimethylchlorosilane, tri-n-propylchlorosilane, tripheylchlorosilane, trimethylsilyliodide, dimethylethoxysilane, dimethylvinylethoxysilane, dimethylvinylmethoxysilane, trimethylethoxysilane, trimethylmethoxysilane and the like. These silane compounds are also commercially available, and for example, KA-31, TESC, TBMS, IIPSC and TES (all are trade names) manufactured by Shin-Etsu Chemical Co., Ltd., Z-6013 (trade name) manufactured by Dow Corning Toray Co., Ltd., and the like can be exemplified.

The reaction of a silylated amino acid/silane compound copolymer and a silane compound represented by the general formula (VIII) can be carried in the same manner as for the reaction of a silylated peptide/silane compound copolymer composition and a silane compound having three alkyl groups bonding to a silicon atom, described in Patent Document 5. Specifically, an aqueous solution of a silylated amino acid/silane compound copolymer composition is stirred at 20 to 100°C, preferably 30°C to 80°C, and a silane compound represented by the general formula (IX) is dropped into this and these are reacted. In the case of a silane compound represented by the general formula (IX) wherein R⁴ is a halogen group, the halogen group is converted into a hydroxyl group by dropping directly into a silylated amino acid/silane compound copolymer aqueous solution, and the resultant hydroxyl group is condensed with a hydroxyl group of the silylated amino acid/silane compound copolymer, while in the case of a silane compound wherein R⁴ is an alkoxy group, it is necessary that
the reaction system is adjusted to pH1 to 4 and the reaction is started, and thereafter, pH is adjusted to around neutrality to conduct the reaction, alternatively,
a compound represented by the general formula (IX) is hydrolyzed previously in an aqueous solution of pH2 to 3 to obtain a silane compound represented by general formula (VIII), and thereafter, the compound is dropped into the above-described silylated amino acid/silane compound copolymer solution to conduct the reaction around neutrality.

Stirring is continued for 1 to 24 hours after completion of dropping of a silane compound represented by the general formula (IX), the solution is neutralized with a sodium hydroxide aqueous solution, a potassium hydroxide aqueous solution or the like, and further, stirring is continued for 1 to 24 hours to complete the reaction. Thus, a silylated amino acid/silane compound copolymer having good stability is obtained.

The silylated amino acid/silane compound copolymer obtained as described above is a silylated amino acid/silane compound copolymer constituting the cosmetic substrate of the first embodiment of the present invention. Specifically, it is a copolymer having a structural unit U represented by the above-described general formula (Ia), (Ib) or (Ic) and a structural unit W represented by the general formula (Id) or (Ie) wherein structural unit W:structural unit U is in the range of 1:5 to 1:150 (molar ratio). In a preferable embodiment, a R³₃Si-O- group is further bonded to Si at the end (or, a R³₃Si- group is bonded to O at the end). It is believed that, in this copolymer, O contained in the structural unit represented by the general formula (Ia), (Ib), (Ic), (Id) or (Ie) and Si in the other structural unit are bonded. Further, a case in which the above-described bond represented by the general formula (If) is contained is also envisaged. That is, this copolymer is a mixture of various structures, but, in a preferable embodiment, this copolymer is mainly composed of a copolymer represented by the following general formula (X): 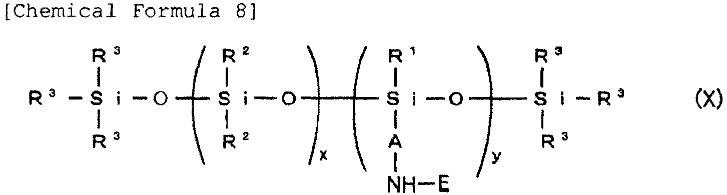 [wherein, R¹, R², R³, A and E are as described above, x and y represent an integer of 1 or more, and x:y = 1:5 to 1:150 is satisfied. In this case, x and y only indicate the presence number of each monomer, and do not represent the order of a sequence]. The degree of polymerization, namely x+y, is preferably about 5 to 1000, more preferably about 10 to 100 to show good adsorbability for hair and skin without imparting stickiness.

### [Cosmetic containing silylated amino acid/silane compound copolymer]

Cosmetic compositions can be prepared by inclusion of a cosmetic substrate composed of a silylated amino acid/silane compound copolymer produced as described above. The cosmetic capable of containing a silylated amino acid/silane compound copolymer includes, for example, a hair rinse, a hair treatment, a hair conditioner, a hair cream, a split hair coating agent, a shampoo, a hair setting agent, a hair color, a permanent wave agent and the like as hair cosmetics, and, for example, a skin cream, a milky lotion, a facial cleanser, a cleansing cream, a skin care gel, a beauty essence and the like as skin cosmetics.

The content of a cosmetic substrate composed a silylated amino acid/silane compound copolymer in the cosmetic of the present invention (blending amount in cosmetic) is preferably about 0.01% by mass to 20% by mass, more preferably 0.1% by mass to 10% by mass, though it varies slightly depending on the kind of the cosmetic such as rinse away type or not rinse away type. When the content of a silylated amino acid/silane compound copolymer is less than the above-described range, there is a possibility that the effect of imparting lustrous gloss, good sliding effect, moisturizing feeling and a split hair preventing effect to hair and the effect of imparting smoothness and moist feeling to skin are not manifested. When over the above-described range, the adhesion amount to hair and skin becomes too large and stickiness is possibly generated, and storage stability of the cosmetic worsens in some cases.

The cosmetic is constituted, as an essential ingredient, of a cosmetic substrate composed of a silylated amino acid/silane compound copolymer as described above, and anionic surfactants, nonionic surfactants, cationic surfactants, ampholytic surfactants, cationic polymers, ampholytic polymers, anionic polymers, thickening agents, animal and plant extracts, polysaccharides or derivatives thereof, hydrolysates of proteins derived from animals, plants or microorganisms and derivatives thereof, neutral or acidic amino acids, moistening agents, lower alcohols, higher alcohols, oils and fats, silicones, various dyes and pigments, antiseptic agents, perfumes and the like can be added to the product in a range wherein the property of the silylated amino acid/silane compound copolymer is not deteriorated.

### EXAMPLES

% described in the following examples and production examples is by mass in all events.

### Production Example 1: Production of N-[2-hydroxy-3-[3'-(dihydroxymethylsilyl)propoxy]propyl]arginine (silylated arginine)

Into a 2-liter beaker was charged 100 g (0.575 mol) of arginine, and 600 mL of water was added thereto. Then the mixture was stirred and a 17% hydrochloric acid aqueous solution was added to adjust pH to 9.0. This solution was heated at 50°C, and 3-glycidoxypropylmethyldiethoxysilane [KBE-402 (trade name) manufactured by Shin-Etsu Chemical Co., Ltd.] (146 g, 0.575 mol, equimolar number to arginine) was dropped into the solution over a period of about 2 hours while stirring. After dropping, stirring at 50°C was continued for 14 hours. Thereafter, a 17% hydrochloric acid aqueous solution was added to adjust pH to 6.0, to obtain 816 g of an aqueous solution of N-[2-hydroxy-3-(3'-dihydroxymethylsilyl)propoxy]propyl]arginine (silylated arginine) having a solid content concentration of 24.6%. The reaction ratio determined from the variation of the amino nitrogen amount before and after the reaction was 82%. The molar number of the produced silylated arginine calculated based on this was 0.448.

The solutions before and after the reaction were subjected to liquid chromatography (hereinafter, referred to as "HPLC") analysis under the conditions mentioned below. As a result, after the reaction, a peak around a molecular weight of 174 of arginine as the raw material nearly disappeared and a new peak was detected around a molecular weight of 366 of silylated arginine. That is, production of silylated arginine could be confirmed.

### [Analysis condition for liquid chromatography (HPLC)]

Separation column: TSKgel G3000PWxL (diameter 7.8 mm - length 300 mm)
Eluent: 0.1% trifluoroacetic acid aqueous solution/acetonitrile = 55/45
Flow rate: 0.3 mL/min
Detector: RI (differential refractive index) detector and UV (ultraviolet) detector, 210 nm
Standard sample: glutathione (Mw 307), bradykinin (Mw 1,060), insulin B chain (Mw 3,496), aprotinin (Mw 6,500)

### Production Examples 2 to 5

A reaction of an amino acid and a silane coupling agent (3-glycidoxypropylmethyldiethoxysilane: KBE-402 manufactured by Shin-Etsu Chemical Co., Ltd.) was conducted in the same manner as in Production Example 1, excepting that arginine was changed to other amino acids shown in Table 1 and the reaction conditions were changed as shown in Table 1. After the reaction, pH was adjusted to values shown in Table 1, to obtain reaction solutions (aqueous solution). The yield and the solid content concentration of this reaction solution, and the reaction ratio determined from the variation of the amino nitrogen amount before and after the reaction (regarding Production example 4 only, the reaction ratio determined from the reduction rate of a peak around a molecular weight of 115), the molar number of the produced silylated amino acid calculated based on this are shown in Table 2.

The solutions before and after the reaction were subjected to HPLC analysis under the same conditions as in Production Example 1. As a result, after the reaction, a peak of an amino acid as the raw material nearly disappeared and a new peak was detected around the position shown in column of "HPLC peak" in Table 2, in any of Production Examples 2 to 5. From the results, productions of silylated amino acids shown in column of "Reaction product" in Table 2 could be confirmed.

| production example No. | amino acid use amount: mol | water use amount: mL | silane coupling agent use amount (ratio to amino acid) | pH of reaction solution | dropping temperature time | reaction temperature time | pH of solution after reaction |
|---|---|---|---|---|---|---|---|
| 1 | Arginine 0.575 | 600 | 0.575 mol (equimolar amount) | 9.0 | 50°C 2 hours | 50°C 14 hours | 6.0 |
| 2 | Lysine 0.148 | 450 | 0.296 mol (double molar amount) | 9.0 | 50°C 1 hour | 50°C 14 hours | 7.0 |
| 3 | Glysine 0.666 | 400 | 0.666 mol (equimolar amount) | 9.7 | 50°C 1 hour | 50°C 14 hours | 6.0 |
| 4 | Proline 0.434 | 400 | 0.434 mol (equimolar amount) | 9.7 | 50°C 1 hour | 50°C 14 hours | 6.0 |
| 5 | Glutamic acid 0.340 | 400 | 0.340 mol (equimolar amount) | 9.8 | 40°C 1 hour | 50°C 14 hours | 6.0 |

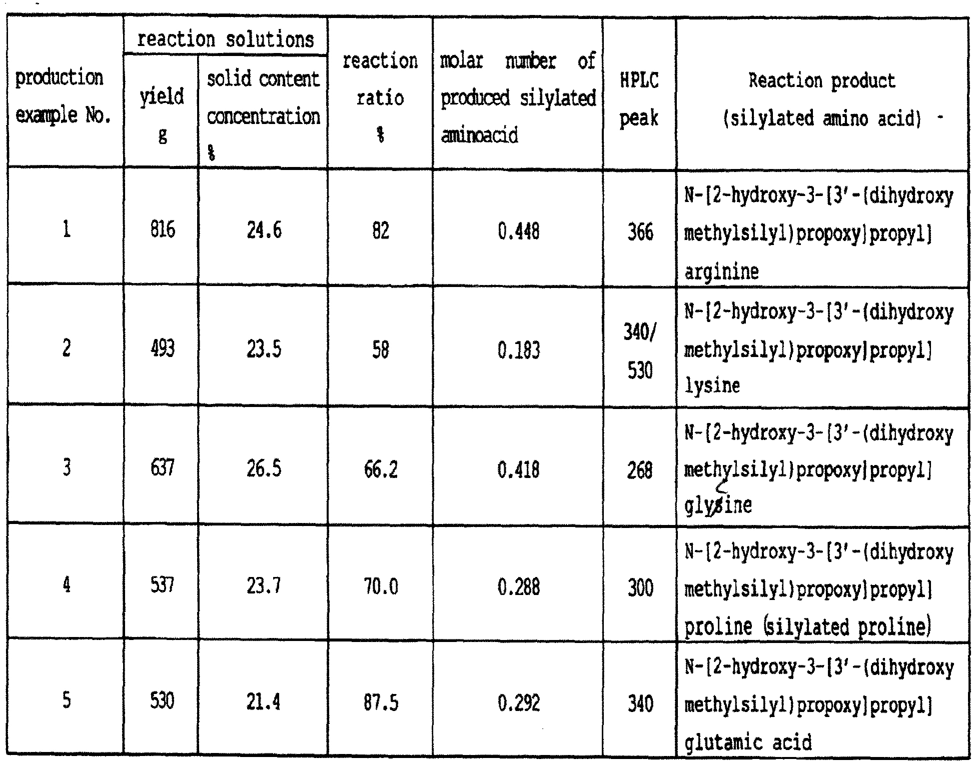

### Example 1: Production of N-[2-hydroxy-3-[3'-(dihydroxymethylsilyl)propoxy]propyl]arginine/dimethyldiethoxy

Into a 1-liter glass round reaction vessel was charged 100 g of the silylated arginine solution (0.055 mol as silylated arginine) obtained in Production Example 1 and water was added to this to adjust the solid content concentration to 20% by mass, then a 17% hydrochloric acid aqueous solution was added to adjust pH to 1.3. This solution was heated at 60°C and a mixed liquid of 122.1 g (0.825 mol) of dimethyldiethoxysilane [KBE-22 (trade name) manufactured by Shin-Etsu Chemical Co., Ltd.] and 227.7 g (0.625 mol) of octyltriethoxysilane [KBE-3083 (trade name) manufactured by Shin-Etsu Chemical Co., Ltd.] was dropped into the solution over a period of 1 hour while stirring. After completion of dropping, stirring at 60°C was continued for 14 hours. Next, a sodium hydroxide aqueous solution was dropped to adjust pH of the solution to 6.0, the liquid temperature was controlled to 40°C and stirring of the liquid was continued for 4 hours, to carry out polycondensation.

Next, 10 g (0.092 mol) of trimethylchlorosilane [KA-31 (trade name) manufactured by Shin-Etsu Chemical Co., Ltd.] was dropped into this solution over a period of 15 minutes and stirred, and pH was adjusted to 6.0 with a 25% sodium hydroxide aqueous solution, and stirring at 80°C was continued for 1 hour. Thereafter, this solution was concentrated under reduced pressure, the generated alcohol was removed, and water was added to adjust the concentration, to obtain 186 g of an aqueous solution of a silylated arginine/silane compound copolymer having a solid content concentration of 75%.

The resultant copolymer was subjected to infrared absorption spectrum (IR) analysis by an ATR method using FT-IR [IR Prestige-21 (trade name)] manufactured by Shimadzu Corp. As a result, a peak derived from Si-CH₃ was detected around 1260 cm⁻¹, a peak derived from Si-O-Si was detected around 1100 to 1000 cm⁻¹ and a peak derived from an alkyl group derived from octyltriethoxysilane was detected around 2960 cm⁻¹. Further, HPLC analysis was performed under conditions shown in Production Example 1, as a result, a peak of silylated arginine around a molecular weight of 366 nearly disappeared. From these results, it could be confirmed that this compound was a silylated arginine/silane compound copolymer.

The viscosity at 20°C of the silylated arginine/dimethyldiethoxysilane/octyltriethoxysilane copolymer (75%) aqueous solution obtained as described above was measured by a B-type viscometer using a rotor 3 at a rotation frequency of 30, to observe a value of 7500 mPa·s.

### Examples 2 to 12

A reaction of a silylated amino acid and a silane compound was conducted in the same manner as in Example 1, excepting that the kind of the silylated amino acid and the reaction conditions were changed as shown in Tables 3-6, to obtain aqueous solutions shown in the columns of product aqueous solution in Tables 3-6, in yields and solid content concentrations shown in the same columns. The resultant aqueous solutions were subjected to infrared absorption spectrum (IR) analysis in the same manner as in Example 1. As a result, a peak derived from Si-CH₃ was detected around 1260 cm⁻¹, a peak derived from Si-O-Si was detected around 1100 to 1000 cm⁻¹, and further, a peak derived from an alkyl group derived from octyltriethoxysilane was detected around 2960 cm⁻¹ other than Example 5.

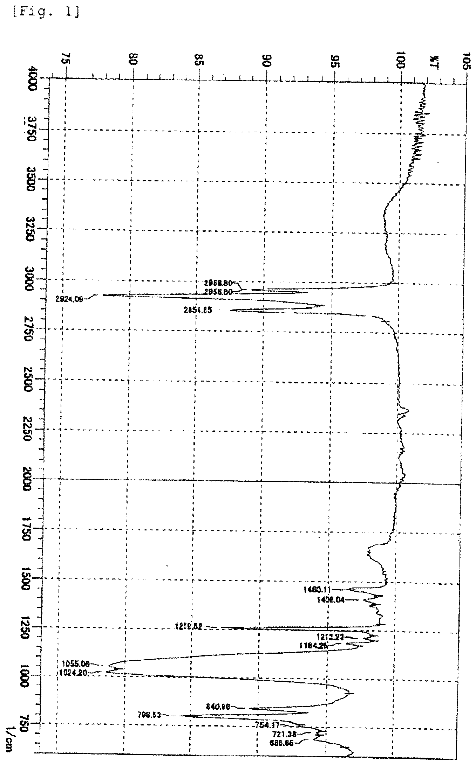

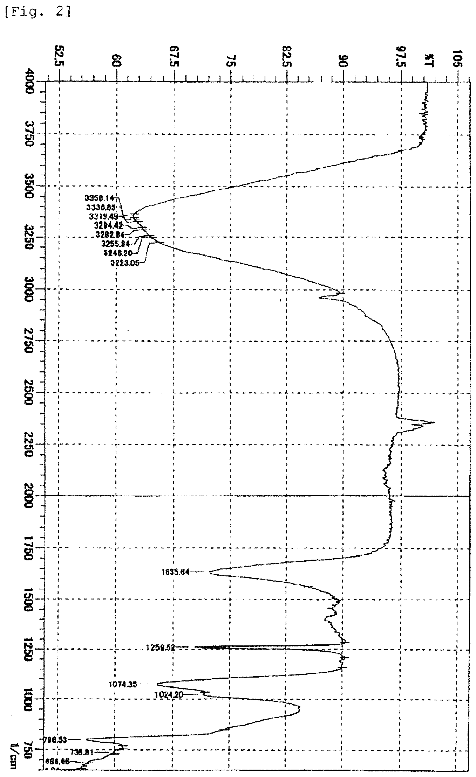

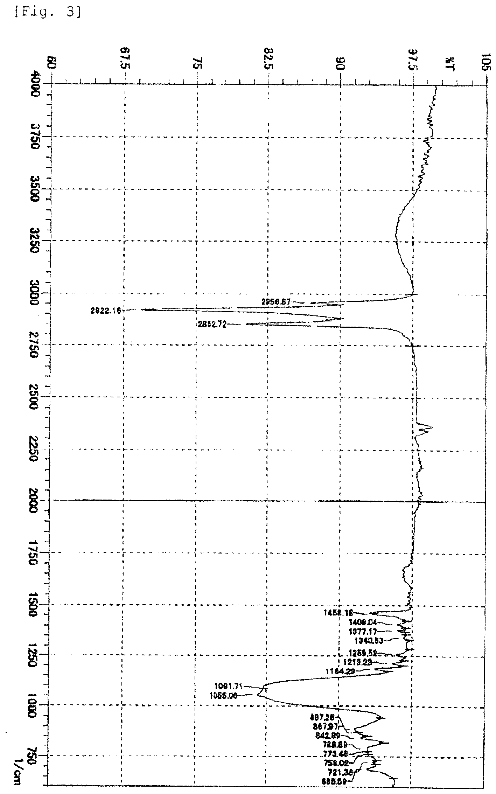

The infrared absorption spectra of the silylated amino acid/silane compound copolymers produced in Examples 1, 5 and 6 are shown in Figs. 1, 2 and 3, respectively.

HPLC analysis was performed under conditions shown in Production Example 1. As a result, a peak of a silylated amino acid as the raw material nearly disappeared. From this, it could be confirmed that the generated compound was a silylated amino acid/silane compound copolymer shown in the column of generated compound in Tables 3-6. Further, the viscosity at 20°C of a 75% aqueous solution of the resultant copolymer was measured by a B-type viscometer using a rotor 3 at a rotation frequency of 30. The results are shown in the column of aqueous solution viscosity in Tables 3-6.

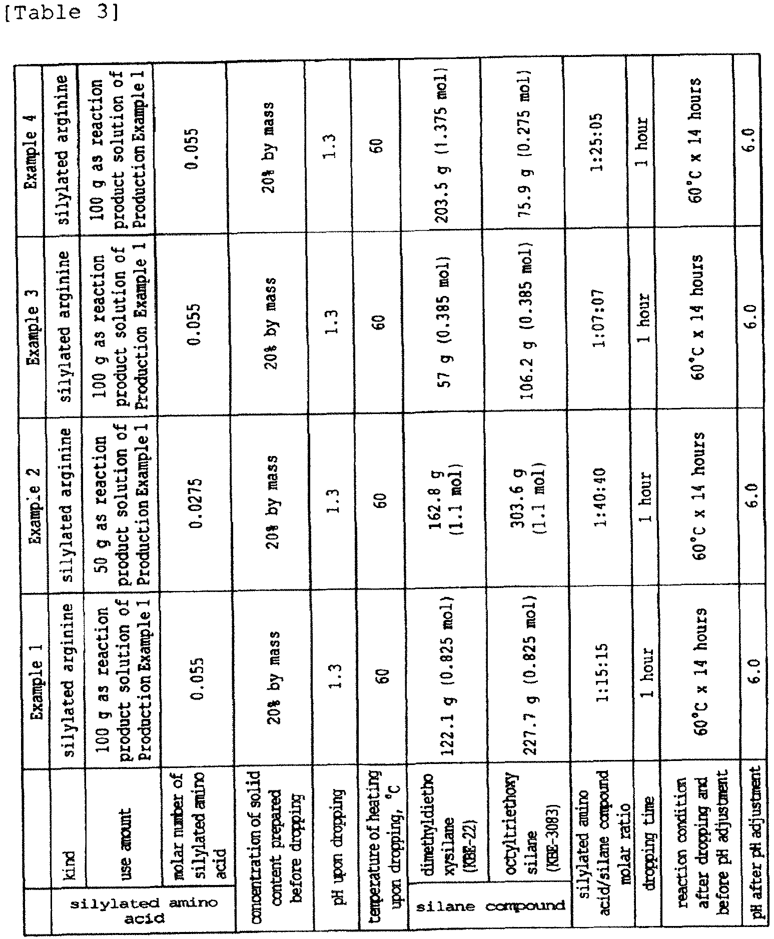

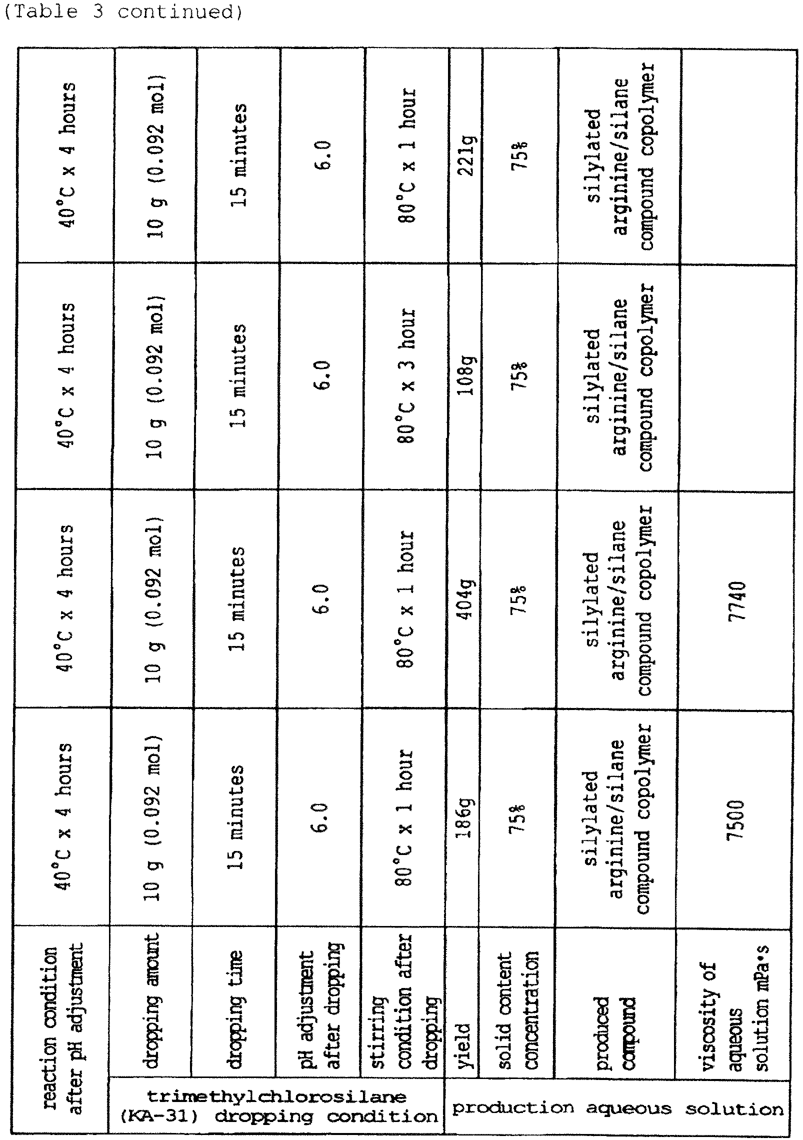

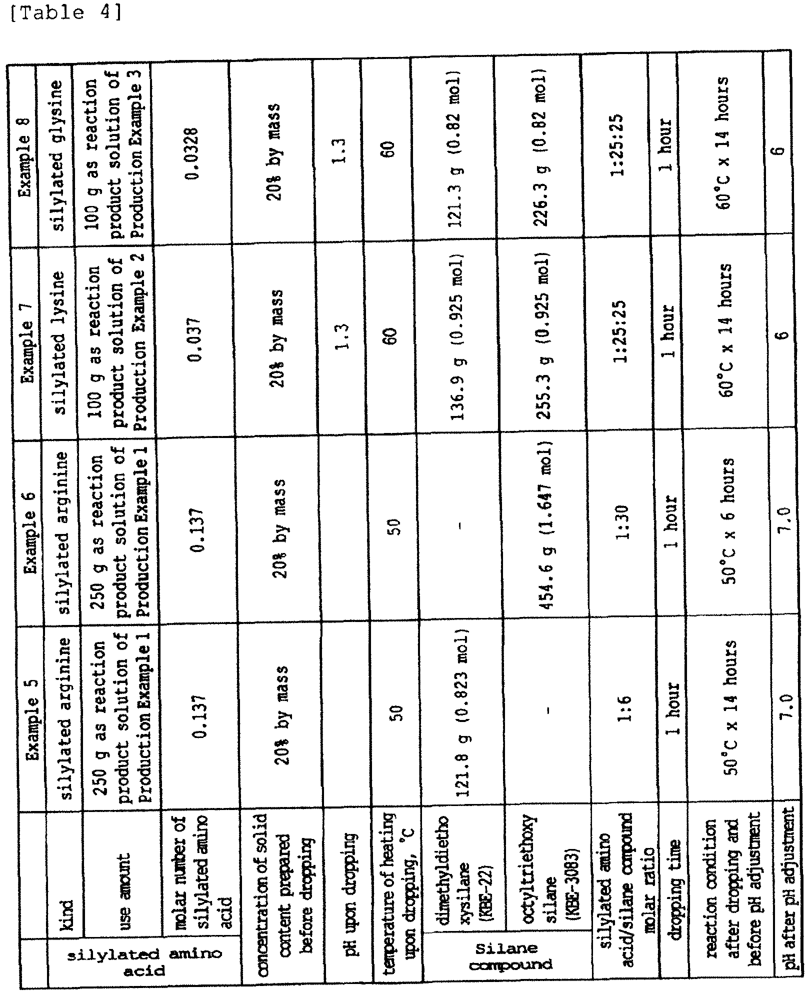

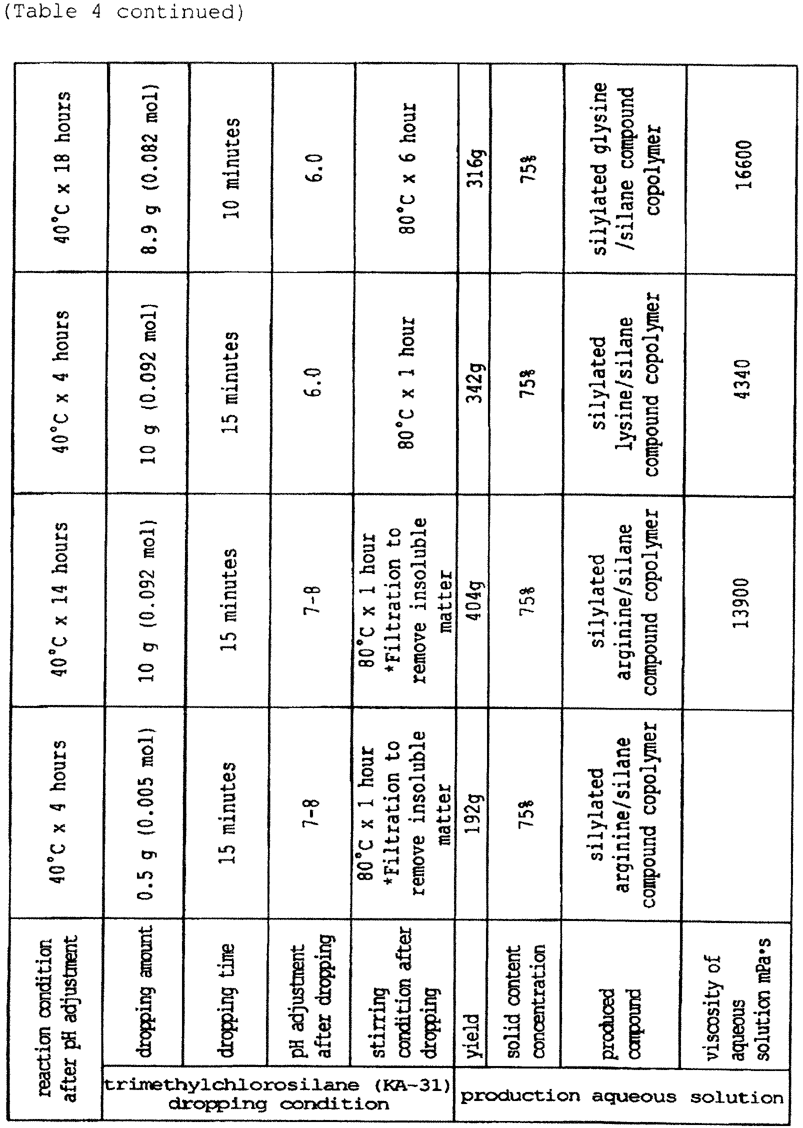

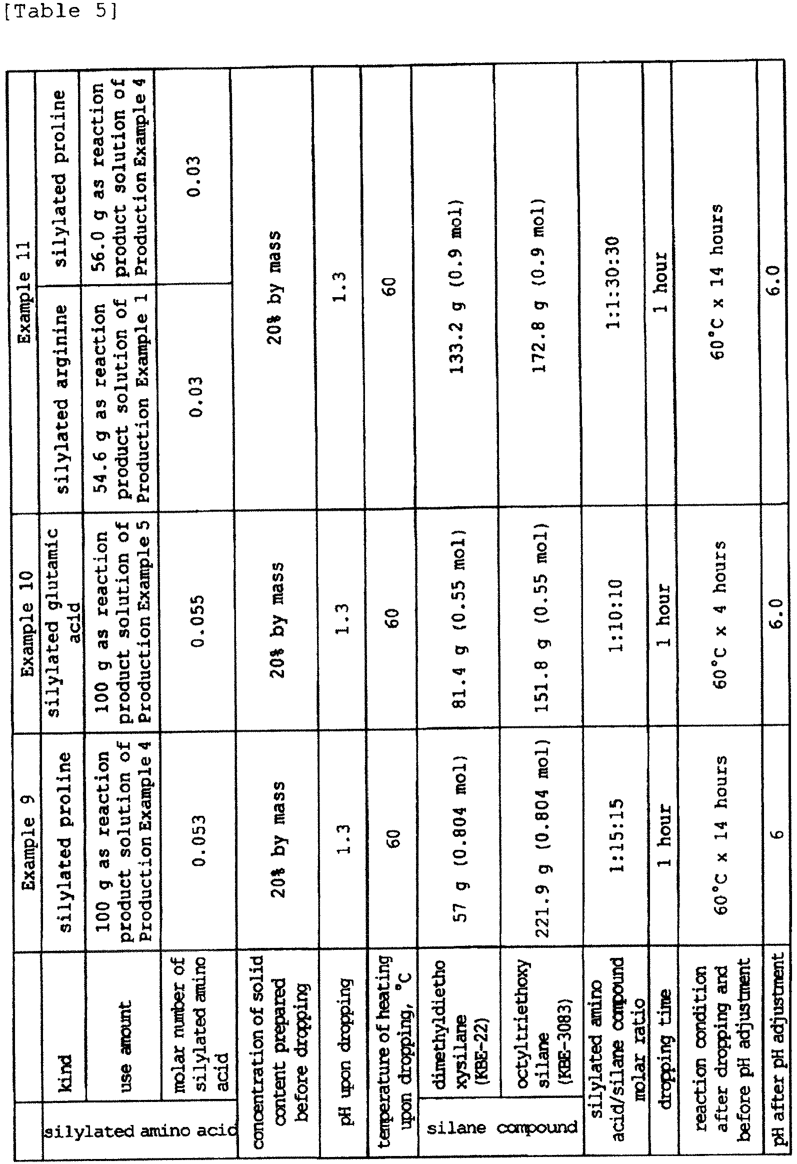

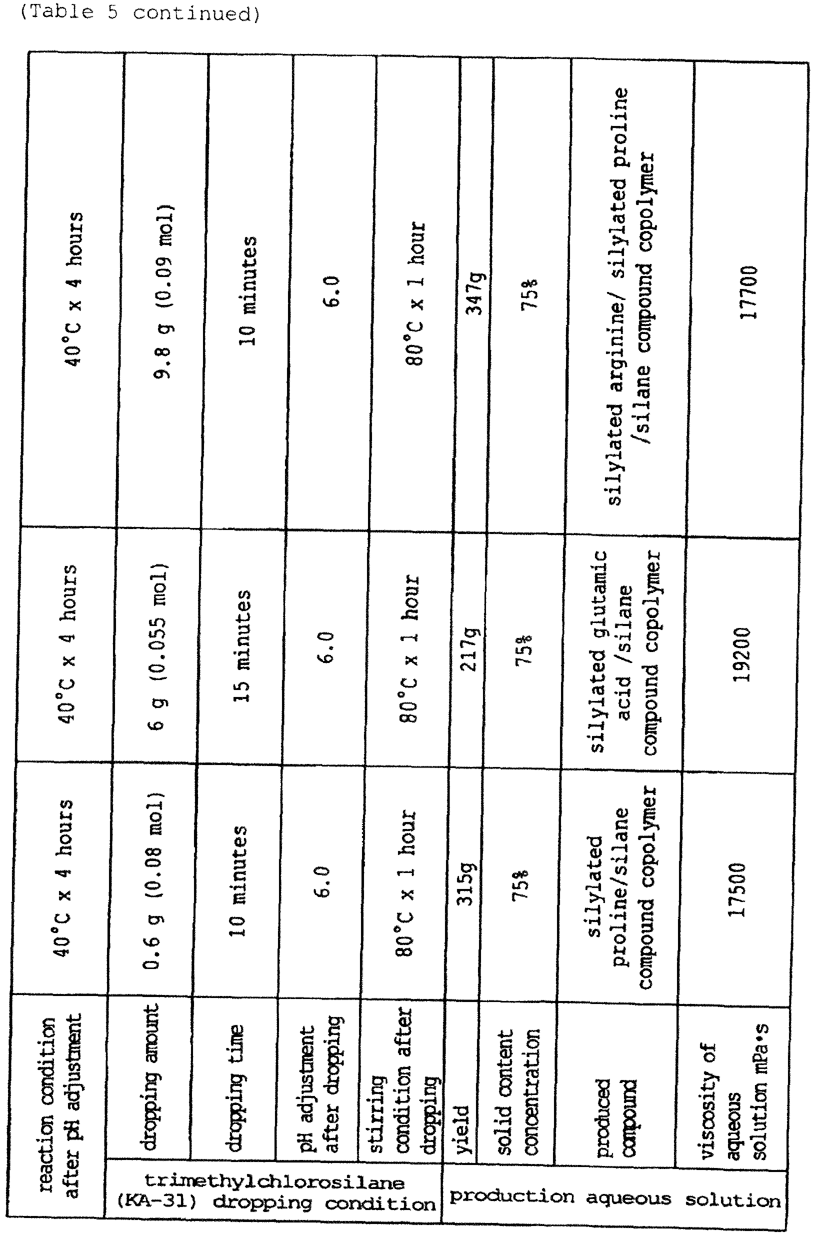

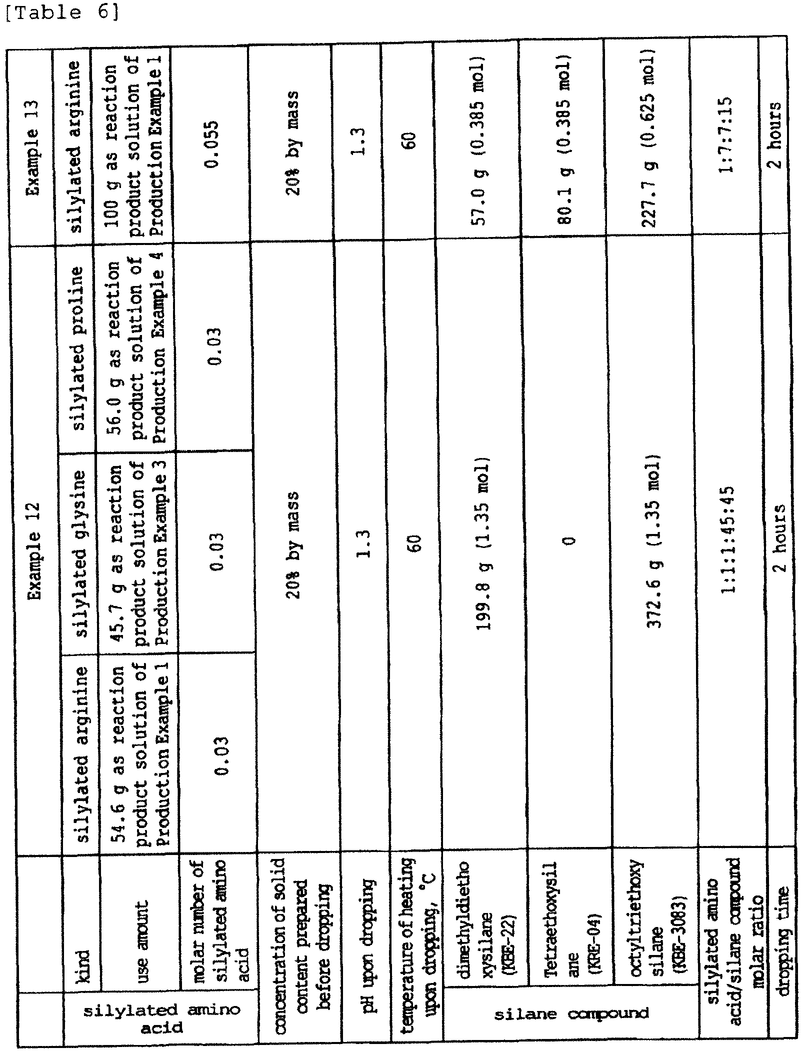

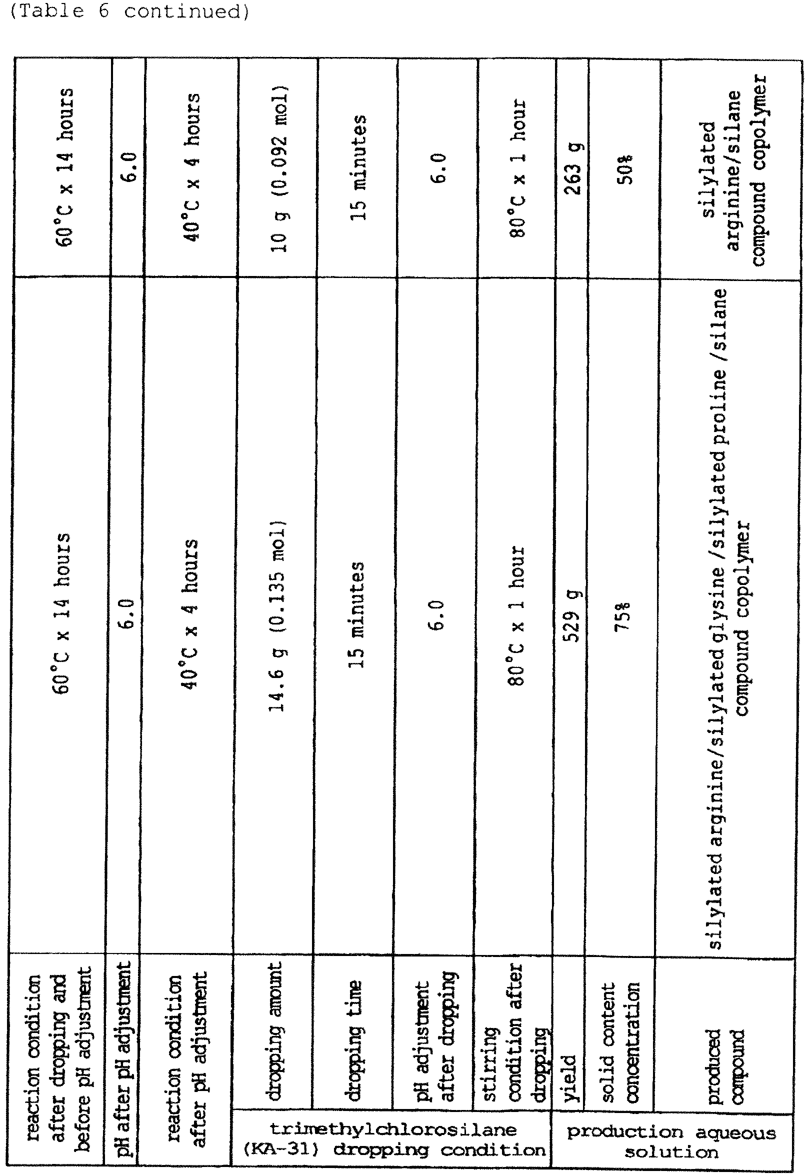

### Reference Example 1: N-[2-hydroxy-3-[3-dihydroxymethylsilyl)propoxy]propyl]casein hydrolysate/dimethyldiethoxysilane/octyltriethoxysilane copolymer (silylated casein hydrolysate/silane compound copolymer) [1:15:15 (molar ratio)]

Into a 1-liter glass round reaction vessel was charged a 25% aqueous solution (200 g, 0.08 mol as molar number obtained from amino nitrogen value) of casein hydrolysate having an average amino acid polymerization degree of 6 determined from the total nitrogen amount and the amino nitrogen amount, and having an average value of the number of acidic amino acids of 1.7, an average value of the number of neutral amino acids of 3.5 and an average value of the number of basic amino acids of 0.8 calculated based on amino acid analysis, and pH was adjusted to 9.5 with a 25% sodium hydroxide solution. This solution was heated at 50°C, and 19.8 g of 3-glycidoxypropylmethyldiethoxysilane (0.08 mol, 1.0 equivalent with respect to amino nitrogen amount of casein hydrolysate) was dropped into the solution over a period of about 1 hour while stirring. After completion of dropping, stirring at 50°C was continued for 14 hours. Thereafter, a 17% hydrochloric acid aqueous solution was added to adjust pH to 6.0, to obtain 214 g of an aqueous solution of silylated casein hydrolysate having a solid content concentration of 22%. The reaction rate determined from the variation of the amino nitrogen amount before and after the reaction was 81%, and the molar number of silylated casein hydrolysate calculated based on this was 0.04.

Next, to this solution was added a 17% hydrochloric acid aqueous solution to adjust pH to 1.3, and a mixture of 88.8 g of dimethyldiethoxysilane (0.6 mol, 15 equivalent with respect to silylated casein hydrolysate) and 165.6 g of octyltriethoxysilane (0.6 mol, 15 equivalent with respect to silylated casein hydrolysate) was dropped into this solution while stirring at 60°C over a period of about 2 hours. After dropping, stirring at 50°C was continued for 14 hours. Then, a 25% sodium hydroxide aqueous solution was dropped to adjust pH of the solution to 6.0, the liquid temperature was controlled to 40°C and stirring was continued for 4 hours, to carry out polycondensation. Next, 6.5 g (0.06 mol) of trimethylchlorosilane was dropped into this solution over a period of 15 minutes and mixed. During this period, a 25% sodium hydroxide aqueous solution was dropped simultaneously to keep pH at 7 to 8. After completion of dropping, stirring was further continued for 3 hours, to complete the reaction. Thereafter, this solution was concentrated under reduced pressure, the generated alcohol was removed, and water was added to adjust the concentration, to obtain 275 g of an aqueous solution of a silylated casein hydrolysate/silane compound copolymer having a solid content concentration of 75%.

Reference Example 2: N-[2-hydroxy-3-[3-dihydroxymethylsilyl)propoxy]propyl] wheat protein hydrolysate/dimethyldiethoxysilane/octyltriethoxysilane copolymer (silylated wheat protein hydrolysate/silane compound copolymer) [1:7:7 (molar ratio)]

Into a 1-liter glass round reaction vessel was charged a 25% aqueous solution (200 g, 0.07 mol as molar number obtained from amino nitrogen value) of wheat protein hydrolysate having an average amino acid polymerization degree of 7 determined from the total nitrogen amount and the amino nitrogen amount, and having an average value of the number of acidic amino acids of 2.7, an average value of the number of neutral amino acids of 3.9 and an average value of the number of basic amino acids of 0.4 calculated based on amino acid analysis, and pH was adjusted to 9.5 with a 25% sodium hydroxide solution. This solution was heated at 50°C, and 17.7 g of 3-glycidoxypropylmethyldiethoxysilane (0.07 mol, 1.0 equivalent with respect to amino nitrogen amount of wheat protein hydrolysate) was dropped into the solution over a period of about 1 hour while stirring. After completion of dropping, stirring at 50°C was continued for 14 hours. Thereafter, a 17% hydrochloric acid aqueous solution was added to adjust pH to 6.0, to obtain 234 g of an aqueous solution of N-[2-hydroxy-3-[3-dihydroxymethylsilyl)propoxy]propyl] wheat protein hydrolysate having a solid content concentration of 22%. The reaction rate determined from the variation of the amino nitrogen amount before and after the reaction was 85%, and the molar number of silylated wheat protein hydrolysate calculated based on this was 0.05.

Next, to this solution was added a 25% sodium hydroxide aqueous solution to adjust pH to 10.5, and a mixture of 51.8 g of dimethyldiethoxysilane (0.35 mol, 7 equivalent with respect to silylated wheat protein hydrolysate) and 96.6 g of octyltriethoxysilane (0.35 mol, 7 equivalent with respect to silylated wheat protein hydrolysate) was dropped into this solution while stirring at 60°C over a period of about 2 hours. After dropping, stirring at 50°C was continued for 14 hours. Then, a 25% sodium hydroxide aqueous solution was dropped to adjust pH of the solution to 6.0, the liquid temperature was controlled to 40°C and stirring was continued for 4 hours, to carry out polycondensation. Next, 7.6 g (0.07 mol) of trimethylchlorosilane was dropped into this solution over a period of 15 minutes and mixed. During this period, a 25% sodium hydroxide aqueous solution was dropped simultaneously to keep pH at 7 to 8. After completion of dropping, stirring was further continued for 3 hours, to complete the reaction. Thereafter, this solution was concentrated under reduced pressure, the generated alcohol was removed, and water was added to adjust the concentration, to obtain 214 g of an aqueous solution of a silylated wheat protein hydrolysate/silane compound copolymer having a solid content concentration of 75%.

Next, test examples and examples as a cosmetic are shown. In tables showing formulations of test examples, examples and comparative example, amounts of the components are all parts by mass. When the amount is not solid content amount, the solid content concentration is shown in parentheses after component name.

### Test Example 1: Test of adsorbability to hair

A polymer shown in the column of "blended polymer" in Table 3 (silylated amino acid/silane compound copolymer produced in Examples 1 to 13) was blended, to prepare a hair treatment agent having a composition in Table 7. Damaged hair was treated with the hair treatment agent prepared, and treatment agent remaining feeling, moisturizing feeling and combability of the hair after treatment were evaluated.

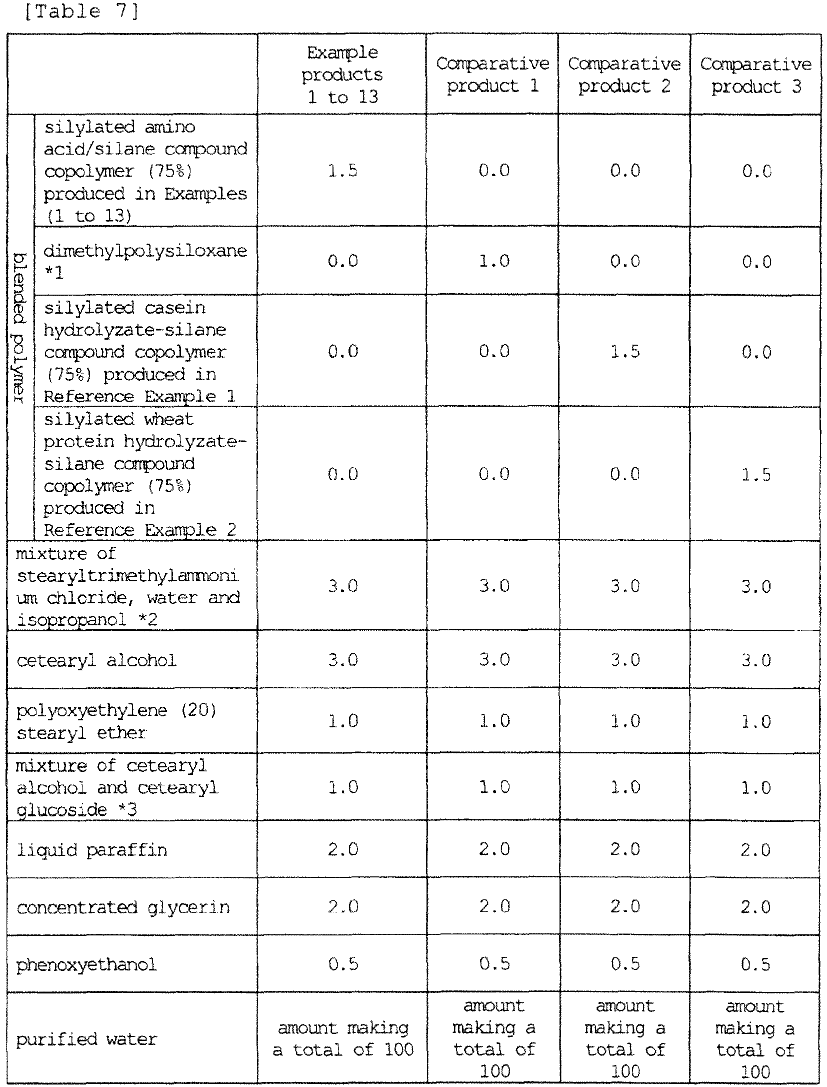

In the Table, *1 denotes KF96A-10cs (trade name) manufactured by Shin-Etsu Chemical Co., Ltd, *2 denotes CATINAL STC-25W (trade name) manufactured by Toho Chemical Industry Co., Ltd. and *3 denotes MONTANOV68 (trade name) manufactured by SEPPIC Co., Ltd.

Prior to the test, hair was washed with a 2% sodium polyoxyethylene (3) lauryl ether sulfate aqueous solution and dried, and a hair bundle of damaged hair having a length of 17 cm and a weight of 2 g was prepared. For making the degree of hair damage of this hair bundle constant, the hair bundle was immersed in a bleach agent prepared by mixing 6% hydrogen peroxide water and 2% ammonia water at a mass ratio of 1:1 at 30°C for 30 minutes, and washed in flowing tap water, then, further rinsed with ion exchanged water and dried by a hair drier. This bleach treatment was repeated 5 times, then, subjected to an adsorbability test described below.

Onto each of the damaged hair bundle produced by the above-described method, 1 g of the hair treatment agent having a composition shown in Table 7 was applied and allowed to show affinity. Then, left in a constant-temperature bath of 40°C for 10 minutes, then, rinsed with hot water of 40°C, and air-dried at room temperature. A hair bundle treated with a treatment agent in which the same amount of water had been added instead of the blended polymer in the composition shown in Table 7 was used as the control, and hair treatment agent remaining feeling, moisturizing feeling and combability were evaluated by 10 panelists according to the following evaluation criteria, and classification was performed according to the total points.

### Evaluation criteria of remaining feeling

3: having high remaining feeling as compared with control
2: having remaining feeling as compared with control
1: not varied from or inferior to control

### Evaluation criteria of moisturizing feeling

3: clear difference of moisturizing feeling is felt as compared with control
2: slight moisturizing feeling is felt as compared with control
1: not varied from or inferior to control

### Evaluation criteria of combability

3: combability is much better and no feeling of caught is felt as compared with control
2: combability is better and feeling of caught is scarcely felt as compared with control
1: not varied from or inferior to control

The evaluation values of panelists for each treatment agent were summed and classified as described below. The results are shown in Table 8. In the following Tables, each of the silylated amino acid/silane compound copolymer produced in Examples 1 to 13 as the test subject is described as "copolymer of Example x" (x is the number of corresponding Example) and the silylated peptide/silane compound copolymer produced in Reference Examples 1 and 2 is described as "copolymer of Reference Example x" (x is the number of corresponding Example).

### Classification of evaluation result

⊚: The total point of 10 panelists is 24 or more
○: The total point of 10 panelists is 17 to 23
Δ: The total point of 10 panelists is 16 or less

**[Table 8]**

| | Blended Polymer | Remaining feeling | Moisturizing feeling | combability |
|---|---|---|---|---|
| Example product 1 | Copolymer of Example 1 | ⊚ | ⊚ | ⊚ |
| Example product 2 | Copolymer of Example 2 | ⊚ | ⊚ | ⊚ |
| Example product 3 | Copolymer of Example 3 | ⊚ | ⊚ | ○ |
| Example product 4 | Copolymer of Example 4 | ⊚ | ⊚ | ⊚ |
| Example product 5 | Copolymer of Example 5 | ⊚ | ⊚ | ○ |
| Example product 6 | Copolymer of Example 6 | ⊚ | ⊚ | ⊚ |
| Example product 7 | Copolymer of Example 7 | ⊚ | ○ | ○ |
| Example product 8 | Copolymer of Example 8 | ○ | ○ | ○ |
| Example product 9 | Copolymer of Example 9 | ○ | ⊚ | ○ |
| Example product 10 | Copolymer of Example 10 | ○ | ○ | ○ |
| Example product 11 | Copolymer of Example 11 | ⊚ | ⊚ | ⊚ |
| Example product 12 | Copolymer of Example 12 | ⊚ | ⊚ | ⊚ |
| Example product 13 | Copolymer of Example 13 | ⊚ | ⊚ | ○ |
| Comparative product 1 | Dimethylpoly siloxane | Δ | Δ | ○ |
| Comparative product 2 | Copolymer of Reference Example 1 | Δ | ○ | Δ |
| Comparative product 3 | Copolymer of Reference Example 2 | Δ | Δ | Δ |

As shown in Table 8, it is apparent that the silylated amino acid/silane compound copolymer produced in each example has better adsorbability to hair as compared with conventional dimethylpolysiloxane and silylated peptide/silane compound copolymer compositions, and can impart excellent moisturizing feeling and good combability to hair.

### Test Example 2: powder dispersibility test

Using the silylated amino acid/silane compound copolymer produced in Examples 1 to 13, a mixture having the blended amount shown in Table 9 was prepared, and whether the power is obtained as an uniform slurry was tested

**[Table 9]**

| | blending amount (g) |
|---|---|
| hydrophobization-treated titanium oxide *4 | 1.0 |
| cyclopentasiloxane *5 | 5.0 |
| silylated amino acid/silane compound copolymer (75%) produced in Examples (1 to 13) | 1.0 |

| | |
|---|---|
| *4 denotes TTO-51 (c) (trade name) manufactured by Ishihara Sangyo Kaisha, Ltd., surface-treated titanium oxide with aluminum hydroxide and stearic acid, and *5 denotes KF-995 (trade name) manufactured by Shin-Etsu Chemical Co., Ltd. | |

In the test, 5.0 g of cyclopentasiloxane was added to 4.0 g of surface-treated titanium oxide and the mixture was stirred, to obtain a semisolid having very high viscosity. Into this, 1.0 g of the silylated amino acid/silane compound copolymer obtained in the example was added and the mixture was stirred, and whether a titanium oxide slurry having low viscosity is obtained was confirmed. Evaluation was performed according to the following evaluation criteria. The results are shown in Table 10.

### Evaluation criteria of powder dispersibility

⊚: a uniform slurry having low viscosity is formed
○: a slurry having low viscosity is formed, but, a few lumps (dumpling-like mass) are observed
Δ: a slurry having low viscosity is formed, but, a lot of lumps are observed
×: the condition of a mixture of titanium oxide and cyclopentasiloxane does not change from the condition when mixed, and a slurry is not obtained

**[Table 10]**

| | powder dispersibility |
|---|---|
| Copolymer of Example 1 | ⊚ |
| Copolymer of Example 2 | ○ |
| Copolymer of Example 3 | ○ |
| Copolymer of Example 4 | ○ |
| Copolymer of Example 5 | ○ |
| Copolymer of Example 6 | ⊚ |
| Copolymer of Example 7 | ○ |
| Copolymer of Example 8 | ○ |
| Copolymer of Example 9 | ○ |
| Copolymer of Example 10 | ⊚ |
| Copolymer of Example 11 | ○ |
| Copolymer of Example 12 | ○ |
| Copolymer of Example 13 | ○ |
| Copolymer of Reference Example 1 | Δ |
| Copolymer of Reference Example 2 | Δ |

As shown in Table 10, all the silylated amino acid/silane compound copolymers of Examples 1 to 13 showed good powder dispersibility.

### Test Example 3: Emulsification test

Using a copolymer shown in Table 11 as an emulsifier and according to the blended amount shown in Table 11, dispersibility of (1) hexyldecyl isostearate, (2) liquid paraffin, (3) olive oil, (4) 2-ethylhexyl methoxycinnamate and (5)dimethylpolysiloxane (5cs) which are oily substances selected from ester oils, hydrocarbons, vegetable oils, oil-soluble ultraviolet absorbers and silicone oils was checked.

**[Table 11]**

| | | Example product | Comparative product |
|---|---|---|---|
| Blended Polymer (emulsi fier) | silylated amino acid/silane compound copolymer (75%) produced in Examples (1 to 13) | 8.0 | 0.0 |
| | silylated peptide/silane compound copolymer (75%) produced in Reference Examples (1 to 2) | 0.0 | 8.0 |
| oily substance | | 70.0 | 70.0 |
| purified water | | 23.0 | 23.0 |

In the test, a silylated amino acid/silane compound copolymer and purified water were mixed and stirred at room temperature by a homo-mixer at 5000 rpm, and an oily substance was dropped into this over a period of 10 minutes. After completion of dropping, stirring was further continued for 10 minutes. The emulsion after preparation was visually observed, and evaluated according to the following criteria. Further, the emulsification test was conducted also on the silylated peptide/silane compound copolymers produced in Reference Examples 1 and 2, instead of the silylated amino acid silane compound copolymers produced in examples, and these were used as comparative products. The results are shown in Table 12.

### Evaluation criteria of emulsion

⊚: a uniform emulsion containing fine particles
○: an approximately uniform emulsion though somewhat coarse particles are observed
Δ: an emulsion is formed directly after preparation, but the emulsification system is broken within 2 weeks

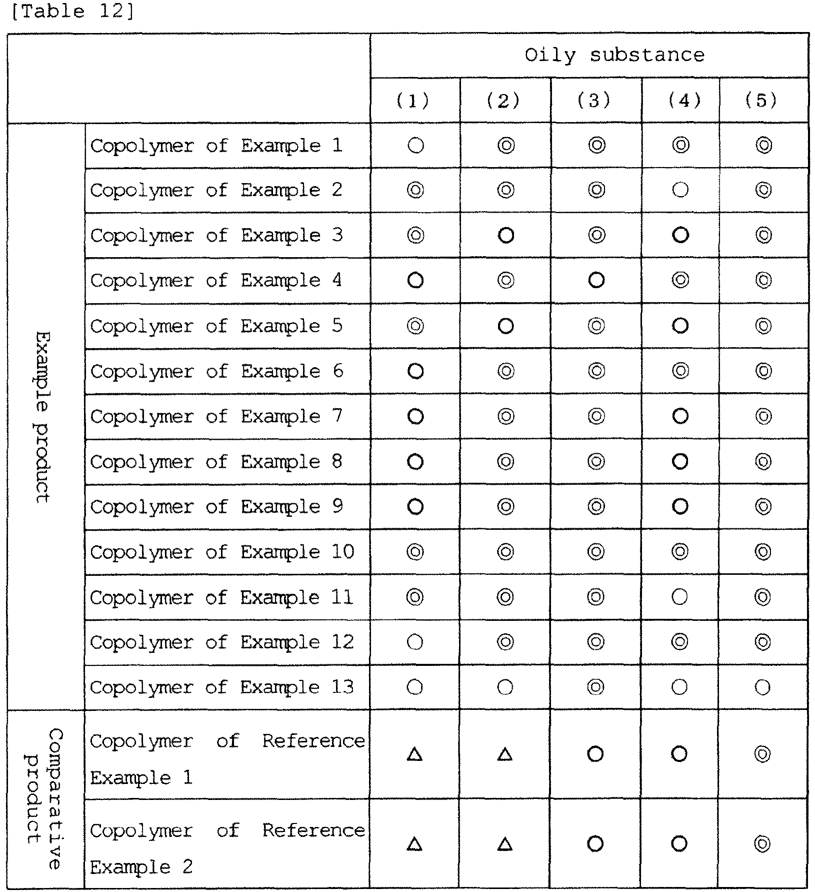

As shown in Table 12, the silylated amino acid/silane compound copolymers of Examples 1 to 13 showed excellent emulsification for all oily substances tested.

### Example 14 and Comparative Examples 1 to 2

Hair conditioners having compositions shown in Table 13 were prepared, and gloss, combability and moisturizing feeling of hair after treatment were evaluated. In Example 14, the silylated arginine/silane compound copolymer (N-[2-hydroxy-3-[3'-(dihydroxymethylsilyl)propoxy]propyl]arginine /dimethyldiethoxysilane/octyltriethoxysilane copolymer [1:15:15 (molar ratio)]) produced in Example 1 was used. In Comparative Example 1, a polyoxyethylene • methylpolysiloxane copolymer was used instead of the silylated arginine/silane compound copolymer was used. In Comparative Example 2, the silylated casein hydrolysate/silane compound copolymer produced in Reference Example 1 was used as the silylated peptide/silane compound copolymer.

**[Table 13]**

| | Example 14 | Comparative example 1 | Comparative example 2 |
|---|---|---|---|
| copolymer of Example 1 (75% aqueous solution) | 3.0 | 0.0 | 0.0 |
| polyoxyethylene•methyl polysiloxane copolymer *6 | 0.0 | 2.3 | 0.0 |
| copolymer of reference example 1 (75% aqueous solution) | 0.0 | 0.0 | 3.0 |
| sodium acrylate/ acryloyldimethyl taurine Na copolymer•isohexadecane• polysorbate 80 mixture *7 | 2.0 | 2.0 | 2.0 |
| 1,3-butylene glycol | 2.0 | 2.0 | 2.0 |
| glycerin | 1.0 | 1.0 | 1.0 |
| liquid paraffin #70 | 1.0 | 1.0 | 1.0 |
| squalane | 2.0 | 2.0 | 2.0 |
| hydrolyzed silk (7%) *8 | 0.5 | 0.5 | 0.5 |
| diethylene glycol monoethyl ether•p-oxybenzoate esters •phenoxyethanol mixture *9 | 0.5 | 0.5 | 0.5 |
| purified water | amount making a total of 100 | amount making a total of 100 | amount making a total of 100 |

In Table 13, *6 denotes KF-6016 (trade name) manufactured by Shin-Etsu Chemical Co., Ltd, *7 denotes SIMULGEL EG (trade name) manufactured by SEPPIC Co., Ltd. and *8 denotes Promois SILK-1000 (trade name) manufactured by SEIWA KASEI Co., Ltd, and *9 denotes Seisept-H (trade name) manufactured by SEIWA KASEI Co., Ltd.

Prior to treatment with the above-described hair treatment agent, hair having washed and bleach-treated was prepared as the hair for treatment. That is, three hair bundles having a length of 13 cm and a weight of 1.7 g were prepared, and previously washed with a 2% sodium polyoxyethylene (3) lauryl ether sulfate aqueous solution, rinsed in flowing tap water and air-dried at room temperature. Next, the bundle was immersed for 30 minutes in a bleach agent prepared by mixing 6% hydrogen peroxide water and 2% ammonia water at 1:1, then, rinsed in flowing tap water, immersed for 5 minutes in a buffer solution (pH 3) of 1 mol/L citric acid and 0.2 mol/L disodium hydrogen phosphate, rinsed in flowing tap water and air-dried at room temperature. This operation was repeated 5 times, and the bundle was subjected to the test.

Hot water of about 40°C was allowed to permeate the bleach-treated hair bundle, then, each 3 g of the hair treatment agents of Example 14 and Comparative Examples 1 to 2 were applied uniformly on each hair bundle and allowed to show affinity to hair, then, left on a wrap film in a constant-temperature bath of 40°C for 10 minutes, and rinsed with hot water, and air-dried at room temperature. Gloss, combability and moisturizing feeling of the hair treated as described above were sensuously evaluated by 10 panelists. In evaluation, the best evaluation is endowed with 2 points, the second evaluation is endowed with 1 point and the bad evaluation is endowed with 0 point, and the average value was used as the evaluation value. The results are shown as the average value of 10 panelists in Table 14.

**[Table 14]**

| | Example 14 | Comparative example 1 | Comparative example 2 |
|---|---|---|---|
| gloss | 1.8 | 0.0 | 1.2 |
| combability | 1.6 | 0.1 | 1.3 |
| moisture-retaining feeling | 2.0 | 0.0 | 1.0 |

As shown in Table 14, hair treated with the hair treatment agent containing the silylated arginine/silane compound copolymer of Example 14 showed high evaluation values in any sensuous evaluation items of gloss, combability and moisturizing feeling of hair, as compared with hair treated with the hair treatment agent of Comparative Example 1 containing the polyoxyethylene•methylpolysiloxane copolymer or the hair treatment agent of Comparative Example 2 containing the silylated casein hydrolysate/silane compound copolymer composition produced in Reference Example 1. The reason for the evaluation value of the hair treatment agent of Example 14 higher than the evaluation value of the hair treatment agent of Comparative Example 2 containing the silylated casein hydrolysate-silane compound copolymer composition produced in Reference Example 1 is guessed that the silylated arginine/silane compound copolymer of Example 14 has an arginine group and thus is easily adsorbed in hair than the casein hydrolysate portion of the silylated casein hydrolysate/silane compound copolymer of Comparative Example 2.

### Example 15 and Comparative Examples 3 to 4

A hair cream having compositions shown in Table 15 was prepared, and used on head hair, then, gloss, combability and moisturizing feeling of the hair were evaluated. In Example 15, the silylated arginine/silane compound copolymer (N-[2-hydroxy-3-[3'-(dihydroxymethylsilyl)propoxy]propyl]arginine /dimethyldiethoxysilane/octyltriethoxysilane copolymer [1:40:40 (molar ratio)]) produced in Example 2 was used. In Comparative Example 3, a poly(oxyethylene • oxypropylene) methylpolysiloxane copolymer was used instead of the silylated arginine/silane compound copolymer was used as a polyether-modified silicone. In Comparative Example 4, the silylated wheat protein hydrolysate/silane compound copolymer produced in Reference Example 2 was used as the silylated peptide/silane compound copolymer.

**[Table 15]**

| | Example 15 | Comparative example 3 | Comparative example 4 |
|---|---|---|---|
| copolymer of Example 2 (75% aqueous solution) | 0.5 | 0.0 | 0.0 |
| Poly (oxyethylene•oxypropyle ne)methyl polysiloxane copolymer *10 | 0.0 | 0.4 | 0.0 |
| copolymer of reference example 2 (75% aqueous solution) | 0.0 | 0.0 | 0.5 |
| Paraffin wax | 10.0 | 10.0 | 10.0 |
| Cetanol | 2.5 | 2.5 | 2.5 |
| Stearic acid | 4.5 | 4.5 | 4.5 |
| Glycerylmonoisostearate | 0.5 | 0.5 | 0.5 |
| 2-ethylhexylpalmate | 15.0 | 15.0 | 15.0 |
| liquid paraffin #350 | 15.0 | 15.0 | 15.0 |
| triethanolamine | 1.8 | 1.8 | 1.8 |
| propyleneglycol | 1.0 | 1.0 | 1.0 |
| diethylene glycol monoethyl ether•p-oxybenzoate esters •phenoxyethanol mixture *9 | 0.5 | 0.5 | 0.5 |
| purified water | amount making a total of 100 | amount making a total of 100 | amount making a total of 100 |

In Table 15, *9 is the same as already mentioned, and *10 denotes SH3749 (trade name) manufactured by Dow Corning Toray Co., Ltd.

Treatment of hair with the above-described hair creams was conducted as described below. That is, three hair bundles having a length of 15 cm and a weight of about 1 g were prepared. The hair bundles were washed with a 2% sodium polyoxyethylene (3) lauryl ether sulfate aqueous solution and rinsed in flowing tap water, then, dried using a drier (cold blast). Each 0.5 g of the hair creams of Example 15, Comparative Examples 3 and 4 were applied on the dried hair bundles while thoroughly spreading the creams, and dried by a hair drier. Gloss, moisture and combability of hair after treatment were evaluated by 10 panelists according to the same evaluation criteria as in Example 14. The results are shown in Table 16. All of the evaluation values are average values.

**[Table 16]**

| | Example 15 | Comparative example 3 | Comparative example 4 |
|---|---|---|---|
| gloss | 1.7 | 0.4 | 0.9 |
| combability | 1.3 | 0.8 | 0.9 |
| moisture-retaining feeling | 1.8 | 0.0 | 1.2 |

As shown in Table 16, hair treated with the hair cream containing the silylated arginine/silane compound copolymer of Example 2 showed higher evaluation values in any evaluation items of gloss, combability and moisturizing feeling of hair, as compared with hair treated with the hair creams of Comparative Example 3 or Comparative Example 4. As apparent from this result, it is apparent that a silylated amino acid/silane compound copolymer is adsorbed successfully in hair and is excellent in the action of imparting gloss, good combability and moisturizing feeling to hair.

### Example 16 and Comparative Examples 5 and 6

Milky lotions having compositions shown in Table 17 were prepared, and affinity to skin, smoothness, moist feeling and little stickiness of skin after application were evaluated. In Example 16, the silylated arginine/silylated proline/silane compound copolymer (N-[2-hydroxy-3-[3'-(dihydroxymethylsilyl)propoxy]propyl]arginine /(N-[2-hydroxy-3-[3'-(dihydroxymethylsilyl)propoxy]propyl]proline/ dimethyldiethoxysilane/octyltriethoxysilane copolymer [1:1:30:30 (molar ratio)]) produced in Example 11 was used. In Comparative Example 5, dimethyl polysiloxane was used instead of the silylated arginine/silylated proline/silane compound copolymer was used. In Comparative Example 6, the silylated casein hydrolysate/silane compound copolymer produced in Reference Example 1 was used as the silylated peptide/silane compound copolymer.

**[Table 17]**

| | Example 16 | Comparative example 5 | Comparative example 6 |
|---|---|---|---|
| copolymer of Example 11 (75% aqueous solution) | 5.00 | 0.00 | 0.00 |
| Methylpolysiloxane *1 | 0.00 | 3.75 | 0.00 |
| copolymer of reference example 2 (75% aqueous solution) | 0.00 | 0.00 | 5.00 |
| Cyclopentasiloxane *5 | 5.00 | 5.00 | 5.00 |
| liquid paraffin | 2.50 | 2.50 | 2.50 |
| squalane | 2.50 | 2.50 | 2.50 |
| Dipropyleneglycol | 1.00 | 1.00 | 1.00 |
| glycerin | 2.00 | 2.00 | 2.00 |
| Carboxyvinylpolymer *11 | 0.10 | 0.10 | 0.10 |
| Potassium hydroxide | 0.02 | 0.02 | 0.02 |
| p-oxybenzoate esters •phenoxyethanol•diethylene glycol ethyl ether mixture *9 | 0.30 | 0.30 | 0.30 |
| purified water | amount making a total of 100 | amount making a total of 100 | amount making a total of 100 |

In Table 17, *1, *5 and *9 are the same as already mentioned, and *11 denotes CARBOPOL980 (trade name) manufactured by Goodrich Corporation.

Affinity to skin when the milky lotions of Example 16 and Comparative Examples 5 to 6 are applied on skin and smoothness, moist feeling and little stickiness of skin after the application were evaluated by 10 panelists according to the same evaluation criteria as in Example 14.

**[Table 18]**

| | Example 16 | Comparative example 5 | Comparative example 6 |
|---|---|---|---|
| Affinity to skin | 1.6 | 0.0 | 1.4 |
| smoothness of skin | 1.0 | 0.2 | 0.8 |
| moist feeling | 1.7 | 0.0 | 1.3 |
| little stickiness | 2.0 | 0.0 | 1.0 |

As apparent from the results shown in Table 18, the milky lotion of Example 16 containing the silylated arginine/silylated proline/silane compound copolymer showed higher evaluation values in any evaluation items, naturally as compared with the milky lotion of Comparative Example 4 containing dimethylpolysiloxane, and even as compared with the milky lotion of Comparative Example 6 containing the silylated hydrolyzed casein/silane compound copolymer.

### Example 17 and Comparative Examples 7 to 8

Three kinds of massage creams were prepared, and spreadability upon application, and moist feeling and little stickiness of skin after application were evaluated. In Example 17, the silylated arginine/silylated glycine/silylated proline/silane compound copolymer (N-[2-hydroxy-3-[3-(dihydroxymethylsilyl)propoxy]propyl]arginine/(N-[2-hydroxy-3-[3-(dihydroxymethylsilyl)propoxy]propyl]glycine/(N-[2-hydroxy-3-[3-(dihydroxymethylsilyl)propoxy]propyl]proline /dimethyldiethoxysilane/octyltriethoxysilane copolymer [1:1:1:45:45 (molar ratio)]) produced in Example 12 was used. In Comparative Example 7, a poly(oxyethylene • oxypropylene) methylpolysiloxane copolymer was used instead of the silylated amino acid/silane compound copolymer was used as a polyether-modified silicone. In Comparative Example 8, the silylated wheat protein hydrolysate/silane compound copolymer produced in Reference Example 2 was used.

**[Table 19]**

| | Example 17 | Comparative example 7 | Comparative example 8 |
|---|---|---|---|
| copolymer of Example 12 (75% aqueous solution) | 2.0 | 0.0 | 0.0 |
| Poly (oxyethylene•oxypropyle ne)methyl polysiloxane copolymer *10 | 0.0 | 1.5 | 0.0 |
| copolymer of reference example 2 (75% aqueous solution) | 0.0 | 0.0 | 2.0 |
| Arachyl glucoside•Arachyl alcohol•Behenyl alcohol mixture *12 | 1.0 | 1.0 | 1.0 |
| Cetearyl glucoside•Cetearyl alcohol mixture *3 | 1.0 | 1.0 | 1.0 |
| Isopropylisostearate | 3.0 | 3.0 | 3.0 |
| Hydrolyzed collagen liquid *13 | 0.1 | 0.1 | 0.1 |
| p-oxybenzoate esters• phenoxyethanol•diethylene glycol ethyl ether mixture*9 | 0.3 | 0.3 | 0.3 |
| Sterilized deionized water | amount making a total of 100 | amount making a total of 100 | amount making a total of 100 |

In Table 19, *3 and *9 are the same as already mentioned, *12 denotes MONTANOV202 (trade name) manufactured by SEPPIC Co., Ltd. and *13 denotes Promois WU-32R (trade name) manufactured by SEIWA KASEI Co., Ltd.

Affinity to skin when the massage creams of Example 17 and Comparative Examples 7 to 8 are applied on hand and smoothness, moist feeling and little stickiness of skin after the application were evaluated by 10 panelists according to the following evaluation criteria. The results are shown as the average value of 10 panelists in Table 20. Evaluation criteria

| | |
|---|---|
| Strongly felt; | 3 point |
| Felt; | 2 point |
| Hardly felt; | 1 point |
| Not felt at all; | 0 point |

**[Table 20]**

| | Example 17 | Comparative example 7 | Comparative example 8 |
|---|---|---|---|
| Affinity to skin | 2.7 | 0.5 | 2.5 |
| smoothness of skin | 2.5 | 1.2 | 2.0 |
| moist feeling | 2.5 | 0.9 | 1.7 |
| little stickiness | 2.0 | 0.6 | 1.0 |

As shown in Table 20, the massage cream of Example 17 showed higher valuation values in any evaluation items of affinity to skin, smoothness, moist feeling and little stickiness of akin after application, naturally as compared with the massage cream of Comparative Example 7 containing the polyether-modified silicone and even as compared with the massage cream of Comparative Example 8 containing the silylated peptide/silane compound copolymer composition. From this result, it is apparent that a silylated amino acid/silane compound copolymer shows good affinity to skin and is excellent in the effect of imparting smoothness and non-sticky moist feeling to skin.

## Claims

1. Use of a silylated amino acid/silane compound copolymer having a structural unit U represented by the following general formula (Ia), (Ib) or (Ic) as an emulsifier or a powder dispersant: 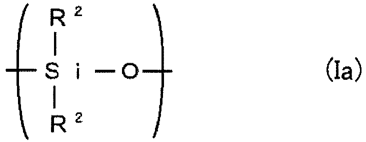 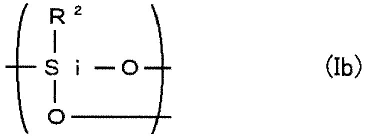 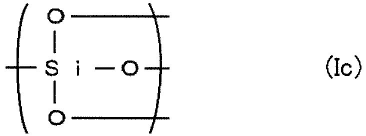 wherein, R² represents a hydroxyl group, a phenyl group, or an alkyl group having 1 to 20 carbon atoms optionally containing nitrogen, sulfur, a halogen or a phenyl group in the group, and the groups R² may be the same or different; and a structural unit W represented by the following general formula (Id) or (Ie): 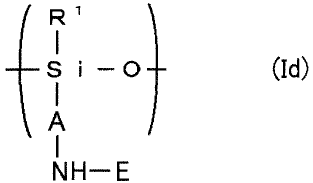 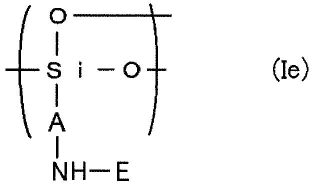 wherein, R¹ represents a hydroxyl group or an alkyl group having 1 to 3 carbon atoms, the groups R¹ may be the same or different, A is a divalent group bonding Si and N, and represents at least one group selected from the group consisting of R^{B}, *R^{B}OCH₂CH(OH)CH₂, *R^{B}S, *R^{B}NH and *R^{B}OCOCH₂CH₂ (R^{B} represents an alkyl group having 1 to 5 carbon atoms, and * represents a side bonding to Si), E represents a residue obtained by removing one primary amino group from an α amino acid, and when E has an amino group other than the α amino group, N in the above-described other amino group may be bonded to A of the other structural unit W], wherein the molar ratio of structural unit W:structural unit U is in the range of 1:5 to 1:150.

2. The use according to Claim 1, wherein the structural unit U is represented by (Ia) or (Ib) and the structural unit W is represented by (Id).

3. The use according to Claim 1 or Claim 2, **characterized in that** the above-described α amino acid contains basic amino acids.

4. The use according to Claim 3 **characterized in that** 40 mol% or more of the above-described α amino acid are basic amino acids.

5. The use according to Claim 3 or Claim 4 in which the basic amino acids are arginine.

6. The use according to any one of Claims 1-5 **characterized in that** a group represented by the following general formula (II) is bonded to the end of the above-described silylated amino acid/silane compound copolymer: 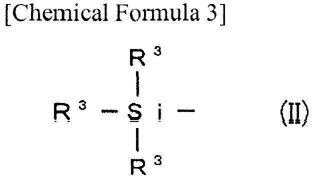 [wherein, R³ represents an alkyl group having 1 to 4 carbon atoms or a phenyl group, and the groups R³ may be the same or different.].

7. Use of a silylated amino acid/silane compound copolymer obtained by poly-condensing at least one silylated amino acid in which a silyl group represented by the following general formula (III) as an emulsifier or powder dispersant: 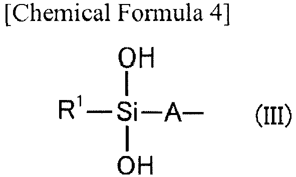 , wherein R¹ represents a hydroxyl group or an alkyl group having 1 to 3 carbon atoms, A is a divalent group bonding Si and N, and represents a group selected from the groups consisting of R^{B}, *R^{B}OCH₂CH(OH)CH₂, *R^{B}S, *R^{B}NH and *R^{B}OCOCH₂CH₂ (R^{B} represents an alkyl group having 1 to 5 carbon atoms, and * represents a side bonded to Si)] is bonded to an α amino group of an α amino acid, and at least one silane compound represented by the following general formula (IV):
R²nSi(OH)ₚY₍₄₋ₚ₋ₙ₎ (IV),
wherein R² represents a hydroxyl group, a phenyl group, or an alkyl group having 1 to 20 carbon atoms optionally containing nitrogen, sulfur, a halogen or a phenyl group in the group, n is an integer of 0 to 2, and n R² may be the same or different, p is an integer of 2 to 4, n+p ≤ 4, and (4-p-n) Y represent an alkoxy group having 1 to 6 carbon atoms or a hydrogen atom, wherein the reaction molar ratio of silylated amino acid:silane compound is in the range of 1:5 to 1:150.

8. The use according to Claim 7 wherein the silylated amino acid/silane compound copolymer is obtainable by poly-condensing the silylated amino acid and the silane compound, then, further reacting a silane compound represented by the following general formula (VIII):
R³₃Si-OH (VIII)
wherein three R³ represent an alkyl group having 1 to 4 carbon atoms or a phenyl group, and the three R³ may be the same or different.

9. Use of a silylated amino acid/silane compound copolymer according to any one of Claims 1-8 in a cosmetic, wherein the cosmetic contains 0.01% by mass or more and 20% by mass or less of the silylated amino acid/silane compound copolymer .

## Patentansprüche

1. Verwendung eines silylierten Aminosäure/Silanverbindungs-Copolymers mit einer Struktureinheit U, dargestellt durch die folgende allgemeine Formel (Ia), (Ib) oder (Ic) als ein Emulgator oder ein Pulverdispergiermittel: 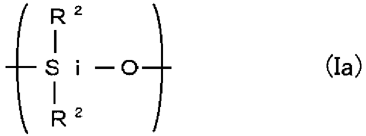 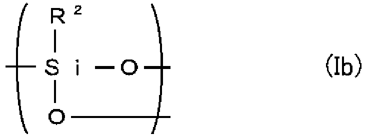 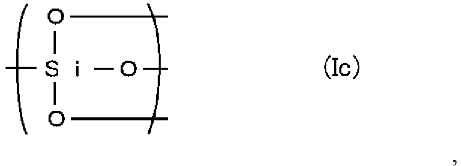 wobei R² eine Hydroxylgruppe, eine Phenylgruppe oder eine Alkylgruppe mit 1 bis 20 Kohlenstoffatomen, die gegebenenfalls Stickstoff, Schwefel, ein Halogen oder eine Phenylgruppe in der Gruppe enthält, darstellt, und die Gruppen R² gleich oder verschieden sein können;
und einer Struktureinheit W, dargestellt durch die folgende allgemeine Formel (Id) oder (Ie): 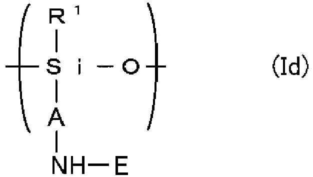 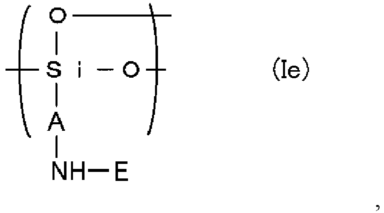 wobei R¹ eine Hydroxylgruppe oder eine Alkylgruppe mit 1 bis 3 Kohlenstoffatomen darstellt, die Gruppen R¹ gleich oder verschieden sein können, A eine zweiwertige Gruppe ist, die Si und N verbindet, und mindestens eine Gruppe darstellt, ausgewählt aus der Gruppe bestehend aus R^{B}, *R^{B}OCH₂CH(OH)CH₂, *R^{B}S, *R^{B}NH und *R^{B}OCOCH₂CH₂ (wobei R^{B} eine Alkylgruppe mit 1 bis 5 Kohlenstoffatomen darstellt, und * eine an Si bindende Seite darstellt), E einen Rest darstellt, erhalten durch Entfernen einer primären Aminogruppe von einer α-Aminosäure und wenn E eine andere Aminogruppe als die α-Aminogruppe hat, kann N in der oben beschriebenen anderen Aminogruppe an A der anderen Struktureinheit W gebunden sein, wobei das Molverhältnis von Struktureinheit W:Struktureinheit U im Bereich von 1:5 bis 1:150 liegt.

2. Die Verwendung gemäß Anspruch 1, wobei die Struktureinheit U durch (Ia) oder (Ib) und die Struktureinheit W durch (Id) dargestellt ist.

3. Die Verwendung gemäß Anspruch 1 oder Anspruch 2, **dadurch gekennzeichnet, dass** die vorstehend beschriebene α-Aminosäure basische Aminosäuren enthält.

4. Die Verwendung gemäß Anspruch 3, **dadurch gekennzeichnet, dass** 40 Mol-% oder mehr der vorstehend beschriebenen α-Aminosäure basische Aminosäuren sind.

5. Die Verwendung gemäß Anspruch 3 oder Anspruch 4, wobei die basischen Aminosäuren Arginin sind.

6. Die Verwendung gemäß einem der Ansprüche 1-5, **dadurch gekennzeichnet, dass** eine Gruppe, die durch die folgende allgemeine Formel (II) dargestellt wird, an das Ende des oben beschriebenen silylierten Aminosäure/Silanverbindungs-Copolymers gebunden ist: 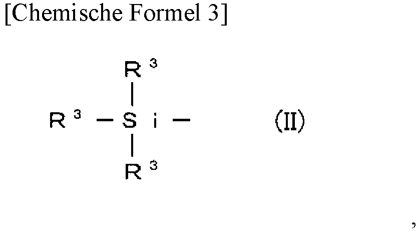 [wobei R³ eine Alkylgruppe mit 1 bis 4 Kohlenstoffatomen oder eine Phenylgruppe darstellt und die Gruppen R³ gleich oder verschieden sein können].

7. Verwendung eines silylierten Aminosäure/Silanverbindungs-Copolymers, erhalten durch Polykondensation mindestens einer silylierten Aminosäure, in der eine Silylgruppe dargestellt durch die folgende allgemeine Formel (III) als ein Emulgator oder ein Pulverdispergiermittel: 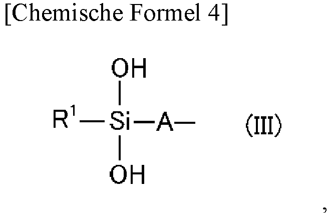
wobei R¹ eine Hydroxylgruppe oder eine Alkylgruppe mit 1 bis 3 Kohlenstoffatomen darstellt, A eine zweiwertige Gruppe ist, die Si und N verbindet, und eine Gruppe darstellt, ausgewählt aus den Gruppen, bestehend aus R^{B}, *R^{B}OCH₂CH(OH)CH₂, *R^{B}S, *R^{B}NH und *R^{B}OCOCH₂CH₂ (wobei R^{B} eine Alkylgruppe mit 1 bis 5 Kohlenstoffatomen darstellt und * eine an Si gebundene Seite darstellt) an eine α Aminogruppe einer α-Aminosäure gebunden ist, und
mindestens einer Silanverbindung, dargestellt durch die folgende allgemeine Formel (IV):
R²nSi(OH)ₚY₍₄₋ₚ₋ₙ₎ (IV),
wobei R² eine Hydroxylgruppe, eine Phenylgruppe oder eine Alkylgruppe mit 1 bis 20 Kohlenstoffatomen die gegebenenfalls Stickstoff, Schwefel, ein Halogen oder eine Phenylgruppe in der Gruppe enthält, darstellt, n eine ganze Zahl von 0 bis 2 ist und n R² gleich oder verschieden sein können, p eine ganze Zahl von 2 bis 4 ist, n+p ≤ 4, und (4-p-n) Y eine Alkoxygruppe mit 1 bis 6 Kohlenstoffatomen oder ein Wasserstoffatom darstellen,
wobei das molare Reaktionsverhältnis von silylierter Aminosäure: Silanverbindung im Bereich von 1:5 bis 1:150 liegt.

8. Die Verwendung gemäß Anspruch 7, wobei das silylierte Aminosäure/Silanverbindungs-Copolymer durch Polykondensation der silylierten Aminosäure und der Silanverbindung, und anschließend weiteres Umsetzen einer Silanverbindung, dargestellt durch die folgende allgemeine Formel (VIII), erhältlich ist:
R³₃Si-OH (VIII),
wobei drei R³ eine Alkylgruppe mit 1 bis 4 Kohlenstoffatomen oder eine Phenylgruppe darstellen und die drei R³ gleich oder verschieden sein können.

9. Verwendung eines silylierten Aminosäure/Silanverbindungs-Copolymers gemäß einem der Ansprüche 1-8 in einem Kosmetikum, wobei das Kosmetikum 0,01 Massen-% oder mehr und 20 Massen-% oder weniger des silylierten Aminosäure/Silanverbindungs-Copolymers enthält.

## Revendications

1. Utilisation d'un copolymère d'acide aminé silylé/composé de silane présentant une unité structurelle U représentée par la formule générale (Ia), (Ib), ou (Ic) suivante comme un émulsifiant ou un dispersant de poudre : 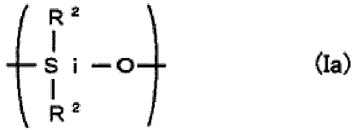 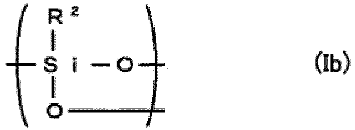 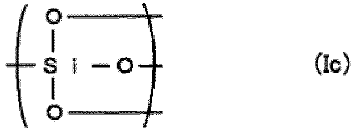 où, R² représente un groupe hydroxyle, un groupe phényle, ou un groupe alkyle ayant de 1 à 20 atomes de carbone contenant éventuellement de l'azote, du soufre, un halogène ou un groupe phényle dans le groupe, et les groupes R² peuvent être identiques ou différents ;
et une unité structurelle W représentée par la formule générale (Id) ou (Ie) suivante : 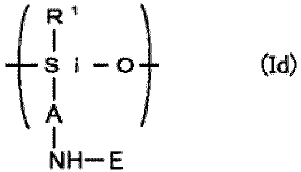 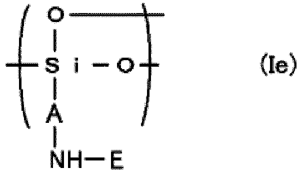 où, R¹ représente un groupe hydroxyle ou un groupe alkyle ayant de 1 à 3 atomes de carbone, les groupes R¹ peuvent être identiques ou différents, A est un groupe divalent liant Si et N, et représente au moins un groupe choisi dans le groupe consistant en R^{B}, *R^{B}OCH₂CH(OH)CH₂, *R^{B}S, *R^{B}NH et *R^{B}OCOCH₂CH₂ (R^{B} représente un groupe alkyle ayant de 1 à 5 atomes de carbone, et * représente une liaison latérale à Si), E représente un résidu obtenu en éliminant un groupe amino primaire d'un acide α-aminé, et lorsque E présente un groupe amino différent du groupe α-amino, N dans l'autre groupe amino décrit ci-dessus peut être lié à A de l'autre unité structurelle W, où le rapport molaire d'unité structurelle W : unité structurelle U se trouve dans l'intervalle de 1:5 à 1:150.

2. Utilisation selon la revendication 1, où l'unité structurelle U est représentée par (Ia) ou (Ib) et l'unité structurelle W est représentée par (Id).

3. Utilisation selon la revendication 1 ou revendication 2, **caractérisée en ce que** l'acide α-aminé décrit ci-dessus contient des acides aminés basiques.

4. Utilisation selon la revendication 3, **caractérisée en ce que** 40 % en mol ou plus de l'acide α-aminé sont des acides aminés basiques.

5. Utilisation selon la revendication 3 ou revendication 4, où les acides aminés basiques sont l'arginine.

6. Utilisation selon l'une quelconque des revendications 1-5, **caractérisée en ce qu'**un groupe représenté par la formule générale (II) suivante est lié à l'extrémité du copolymère d'acide aminé silylé/composé de silane décrit ci-dessus : 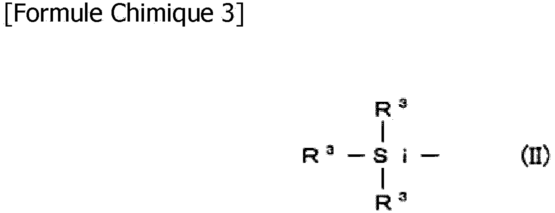 [où, R³ représente un groupe alkyle ayant de 1 à 4 atomes de carbone ou un groupe phényle, et les groupes R³ peuvent être identiques ou différents].

7. Utilisation d'un copolymère d'acide aminé silylé/composé de silane obtenu par polycondensation d'au moins un acide aminé silylé dans lequel un groupe silyle représenté par la formule générale (III) suivante comme un émulsifiant ou dispersant de poudre : 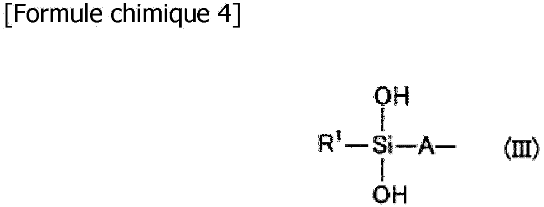 où,
R¹ représente un groupe hydroxyle ou un groupe alkyle ayant de 1 à 3 atomes de carbone, A est un groupe divalent liant Si et N, et représente un groupe choisi parmi les groupes consistant en R^{B}, *R^{B}OCH₂CH(OH)CH₂, *R^{B}S, *R^{B}NH et *R^{B}OCOCH₂CH₂ (R^{B} représente un groupe alkyle ayant de 1 à 5 atomes de carbone, et * représente un côté lié à Si) est lié à un groupe α-amino d'un acide α-aminé, et
au moins un composé de silane représenté par la formule générale (IV) suivante :
R²nSi(OH)ₚY₍₄₋ₚ₋ₙ₎ (IV)
où,
R² représente un groupe hydroxyle, un groupe phényle, ou un groupe alkyle ayant de 1 à 20 atomes de carbone contenant éventuellement de l'azote, du soufre, un halogène ou un groupe phényle dans le groupe, n est un nombre entier de 0 à 2, et les n R² peuvent être identiques ou différents, p est un nombre entier de 2 à 4, n+p ≤ 4, et les (4-p-n)Y représentent un groupe alcoxy ayant de 1 à 6 atomes de carbone ou un atome d'hydrogène,
où le ratio molaire de réaction d'acide aminé silylé : composé de silane se trouve dans l'intervalle de 1:5 à 1:150.

8. Utilisation selon la revendication 7, où le copolymère d'acide aminé silylé/composé de silane peut être obtenu par polycondensation de l'acide aminé silylé et du composé de silane, puis, réaction supplémentaire d'un composé de silane représenté par la formule générale (VIII) suivante :
R³₃Si-OH (VIII)
où,
les trois R³ représentent un groupe alkyle ayant de 1 à 4 atomes de carbone ou un groupe phényle, et les trois R³ peuvent être identiques ou différents.

9. Utilisation d'un copolymère d'acide aminé silylé/composé de silane selon l'une quelconque des revendications 1-8 dans un cosmétique, où le cosmétique contient 0,01 % en masse ou plus et 20 % en masse ou moins du copolymère d'acide aminé silylé/composé de silane.
